(19) **Europäisches Patentamt / European Patent Office / Office européen des brevets**

(11) **EP 2 910 243 A1**

(12) # EUROPEAN PATENT APPLICATION
published in accordance with Art. 153(4) EPC

(43) Date of publication:
26.08.2015 Bulletin 2015/35

(21) Application number: 13847305.3

(22) Date of filing: 16.10.2013

(51) Int Cl.:
*A61K 9/36* (2006.01)   *A61K 9/32* (2006.01)
*A61K 9/22* (2006.01)

(86) International application number:
**PCT/CN2013/001256**

(87) International publication number:
**WO 2014/059748 (24.04.2014 Gazette 2014/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: 16.10.2012  CN 201210393773

(71) Applicant: **Shanghai Institute Of Materia Medica Chinese Academy of Sciences**
**Pudong New District**
**Shanghai 201-203 (CN)**

(72) Inventors:
• **ZHANG, Jiwen**
**Shanghai 201203 (CN)**

• **WU, Li**
**Shanghai 201210 (CN)**
• **YIN, Xianzhen**
**Shanghai 201210 (CN)**
• **GUO, Zhen**
**Shanghai 201210 (CN)**
• **LI, Haiyan**
**Shanghai 201210 (CN)**

(74) Representative: **Kling, Simone**
**Lavoix Munich**
**Bayerstrasse 83**
**80335 München (DE)**

(54) **PHARMACEUTIC OSMOTIC PUMP PREPARATION**

(57)   Provided in the present invention is a pharmaceutic osmotic pump preparation comprising a tablet core, wherein the tablet core contains drugs (or pharmaceutically active ingredients) and materials forming a chamber structure; and a coating film coating the tablet core where a drug release hole is set; wherein when the pharmaceutic osmotic pump preparation releases drugs in water or an aqueous medium, the materials forming the chamber structure lead to the formation of a superfine chamber structure in the coating film, and the total volume of the superfine chamber structure Vb gradually increases over the releasing time, but the releasing rate R (or release amount A) of the pharmaceutically active ingredients is proportional to Vb. The osmotic pump preparation of the present invention is suitable for water-soluble drugs and water-insoluble drugs, and especially is suitable for the controlled release of low solubility drugs or pH dependent drugs.

**Description**

**Technical Filed**

The present invention relates to the pharmaceutic preparation field, particularly to a novel pharmaceutic osmotic pump preparation with special internal micro chamber structures. Said osmotic pump preparation with novel type of structure is suitable for the controlled administration of water soluble drugs and water insoluble drugs, typically of drugs with low solubility.

**Background**

Osmotic pump preparation is characterized with its constant speed of drug releasing, thus, it can avoid the blood concentration fluctuation phenomenon after the administration of conventional preparations and reduce the gastrointestinal and systemic side effects. The release characteristics are less affected by the variable factors of gastrointestinal tract. Different macrostructures of osmotic pump and applications thereof are getting rapid developed and valued. Hundreds of domestic and foreign patents emphasized on the macrostructure of osmotic pump and a variety of macroscopic structures were designed. However, neither internal micro structure of the osmotic pump or its dynamic change is used to control the drug release.

The literature about osmotic pump preparation was first reported in 1955. The earliest administration device by using osmotic pressure as releasing power is Rose-Nelson osmotic pump. Said device is composed of six parts: drug chamber, salt chamber, water chamber, rigid semi-permeable film between water chamber and salt chamber, elastic diaphragm between salt chamber and drug chamber, and rigid film for device covering. It does not have any practical value for human body due to its complicated process and huge volume up to 80 cm$^3$. Higuchi and Leeper improved it and a Higuchi-Leeper osmotic pump was designed in 1971. Water chamber was removed and water in the body was used directly, thereby greatly simplifying the structure of osmotic pump device. In 1974, a elementary osmotic pump was developed by Theeuwes and Alza Corp. US together, which is composed of a tablet core covered with a semi-permeable film and a pore for drug releasing on its coating film(drug releasing pore), thereby simplifying the osmotic pump preparation into a form of common coating tablets and hence suitable for industrial production.

In the 1980's, a new type of osmotic pump was designed for osmotic pump system, i.e., micro-porous osmotic pump. High portion of water soluble component in the controlled releasing film enhanced the permeability of coating film and changed the semi-permeable film into micro-porous film which was permeable to drug molecules. Water soluble components were dissolved after contacting with water, thereby turning the controlled releasing film into micro-porous film. The punching process of osmotic pump preparation was removed for micro porous osmotic pump. However, the presence of enormous pores on the coating film enhanced the diffusion effect and thus resulting zero-order releasing curve turning to first-order. As to slightly soluble or easily soluble drugs, it is very hard to achieve the desired releasing rate only depending on the permeability of the drugs themselves. Therefore, a power layer is added supplementary to the single-layer osmotic pump and drug releasing rate is hence controlled by modulating the swelling rate of the power layer. A push-pull osmotic pump in 1982, a double-layer tablet coated with semi-permeable film, was suitable for easily soluble or slightly soluble drugs. The upper layer of the push-pull osmotic pump is a drug layer composing drugs and auxiliary materials and the lower layer is a power layer composing polymers and permeable materials. The drug layer connects the environment through the releasing pore. After the preparation has been taken, drugs in the drug chamber are powered by polymers in the power layer and then released through the releasing pore. The nifedipine controlled releasing tablet developed by Bayer DE belongs to this kind of double-layer osmotic pump tablet. Many improvements were made based on this osmotic pump and a variety of formulation characterized with different macrostructure were designed or developed, such as Delayed Release System (US5221278) (for pulsatile-released formulation of delayed-released formulation), Piston System(US6132420) (allows good delayed and pulsed effect) etc. However, the industrial application of push-pull osmotic pump is limited since the drug layer needs further recognition besides punching. In 1991, liquid oral osmotic pump system for oral administration was developed, and liquid drugs can be prepared into osmotic pump, including soft capsule liquid osmotic pump (US7338663), hard capsule liquid osmotic pump and time-delayed liquid osmotic pump (US20036596314). Drug liquid is wrapped in the soft capsule, coated with, in turn, isolated layer, permeation enhancing layer, and controlled releasing film layer with releasing pore through out those three layers. After the system is contacted with outside water environment, water permeates through controlled release film, permeation layer swells by water absorption and drug is released through small pores.

Recently, new designs continually appeared based on the known macrostructures of osmotic pumps. For example, the sandwich-type osmotic pump tablet system by using nifedipine as model drug was developed (L. Liu, et al. J Control. Release, 2000, 68: 145-156). The core consists of power layer in the middle and two adhering drug layers and is coated with a semi-permeable film. Advantage of this system is that drug can release through the pores on the opposite sides, thereby avoiding the drug-induced stimulation to the gastrointestinal mucosa. Sandwich osmotic pump tablet allows the omission of the reorganization process to the drug layer. Two-layered mixed pore osmotic pump preparation (D. Prabakaran, et al. Int. J. Pharm., 2004, 284: 95-108), in which single pore is used for upper layer drug releasing and controlled pore is used for lower layer drug releasing, thereby facilitating a simultaneous releasing of drugs with different solubility. The single-layer osmotic pump tablet is further developed into a system with extrudable core for delivering active ingredients of drugs with low solubility in high doses. An asymmetry film composing an extremely thin and rigid surface and thick spongy porous matrix layer is used in a controlled preparation of asymmetry film osmotic pump (US4008719, US6899887), which well solved the problem of incomplete releasing of insoluble drugs in osmotic pump controlled preparation by its high permeability to water and modulated the film permeability to water by controlling the structure and porosity of the film.

In pharmaceutics, pore-forming agent generally refers to the auxiliary material which is added into coating solution for improving the permeability of coating film, thereby improving the drug through-put rate through the film. It hasn't been reported that bubble-shaped structure can be formed when this kind of pore-forming agent is directly used in the core of the tablets. US patent (US4203439, US4331728) reported that materials capable of forming bubbles can be used as power chamber in the core to allow complete release of the drugs at a constant speed. However, the structure of this osmotic pump enables pressured power by bubble, while no vesicular micro structure formed by liquid vesicle is involved.

In conclusion, it is still unsatisfied despite of the osmotic pumps with different structures. Therefore, developing new osmotic pumps with simple structure and better controlled release effects is still in need in this filed.

## Summary of the Invention

The object of the present invention is to provide an osmotic pump preparation characterized with novel structures, wherein, there are dynamic chamber structures of micro bubbles, pores or vesicles, and a method for modulating releasing characteristics of osmotic pump preparation of this structure and a method for determining micro structures are also provided.

The first aspect of the invention provides a pharmaceutical osmotic pump preparation comprising:

a core, comprising drug(s) (or drug active ingredient(s)) and chamber structure forming material(s); and
a coating film for covering the core with drug releasing pore(s) set on said coating film;

wherein, when the drug osmotic pump preparation is used for drug release in water or aqueous medium, micro chamber structures are thus formed inside the coating film by the chamber structure forming materials and total volume of the micro chamber structures Vb increases with releasing time, and releasing rate R of the drug active ingredients (or releasing amount A) is directly proportional to Vb.

In another preferred embodiment, ratio between maximum value of Vb, namely Vbmax and core volume Va is 90-100: 100, preferably 95-100: 100.

In another preferred embodiment, ratio between maximum value of Vb, Vbmax and internal volume of coating film Vc (or a profile volume of osmotic pump shaped with coating film Vm) is 90-100: 100, preferably 95-100: 100.

In another preferred embodiment, the core volume Va equals to the internal volume of coating film Vc or profile volume of osmotic pump shaped with coating film Vm, that is, Va = Vc = Vm.

In another preferred embodiment, a power layer structure is not contained in the osmotic pump shaped with the coating film.

In another preferred embodiment, the term "with drug releasing pore(s) set on said coating film" includes a preformed drug releasing pore located on the coating film, or allowing an agent (such as pore forming agent) capable of forming drug releasing pore to be contained in the coating film, thereby forming a drug releasing pore for drug releasing when the drug osmotic pump preparation contacts with water or aqueous medium.

In another preferred embodiment, said "releasing rate R (or releasing amount A) is directly proportional to Vb" refers to "the correlation coefficient between release rate R (releasing amount A) and Vb is ≥0.9; preferably is ≥0.95".

In another preferred embodiment, said micro chamber structures are liquid chamber structures.

In another preferred embodiment, said micro chamber structures include micro bubble-shaped structures, micro porous structures, micro vesicular structures, or the combination thereof.

In another preferred embodiment, when drug osmotic pump preparation releases drug in water or aqueous medium, the total amount of micro chamber structure rises from to to maximum value Nmax, and then declines.

In another preferred embodiment, said Nmax ≥5000, preferably ≥10000, ≥50000, ≥100000, 500000, 1000000.

In another preferred embodiment, when drug osmotic pump preparation releases drug in water or aqueous medium, the total amounts of micro chamber structures remains unchanged or decreases, and the average volume of said micro chamber structures increases with the releasing time gradually.

In another preferred embodiment, most of (>50%, preferably>70%, >80%) micro chamber structures have a

size of $1\times10^{-6}$-1 mm$^3$

In another preferred embodiment, at least ≥5000, preferably ≥10000 of micro chamber structures have a size of $1\times10^{6}$-1 mm$^3$.

In another preferred embodiment, during releasing process, said micro chamber structures fuse or merge for forming micro chamber structures with lager volumes, thereby forming chamber structures (Generally, although a "micro chamber structure" can be used when the volume is over 10 mm$^3$, the fused chamber structures with larger volume are called chamber structures for convenience.).

In another preferred embodiment, the total volume of said micro chamber structures Vb includes the volume of micro chamber structure, and the volume of chamber structure formed by two or more micro chamber structures.

In another preferred embodiment, the weight of said drug osmotic pump preparation is 5-5000 mg, preferably 10-2000 mg.

In another preferred embodiment, the volume of said drug osmotic pump preparation is 0.05-5000 cm$^3$, preferably 10-2000 cm$^3$.

In another preferred embodiment, said core further comprises one or more components selected from a group consisting of chamber structure shape-modulating agent, lubricating agent, and other pharmaceutical acceptable auxiliary materials or carriers.

In another preferred embodiment, said pharmaceutical acceptable auxiliary materials or carriers include one or more selected from a group consisting of binders, opacifier, filling agent, colorant, antioxidant and osmotic pressure promoting agent.

In another preferred embodiment, the amount(s) of said drug releasing pore is one or more.

In another preferred embodiment, the diameter of said drug releasing pore is 0.01-3 mm, preferably 0.1-2 mm, more preferably 0.5-1.2 mm.

In another preferred embodiment, said coating film contains pore forming agent, and said pore forming agent is used for forming water permeable micro pore on said coating film.

In another preferred embodiment, said coating film contains pore forming agent, and said pore forming agent is used for forming micro pore permeable to water and drugs on said coating film (in this case, micro pore is also named as drug releasing pore).

In another preferred embodiment, said core contains the following components:

0.2-99%, preferably 1-60 wt% of drug(s);
15 - 99.8 wt%, preferably 20-95wt % , more preferably 30-90 wt% of chamber structure forming material(s);
0-50wt%, preferably 1-50wit% of chamber structure shape-modulating agent(s);
0-10wt%, preferably 0.5-10 wt% of lubricating agent(s); and
0-60wt%, 1-60 wt% of pharmaceutical acceptable auxiliary material(s); wherein the content is based on total weight of the core.

In another preferred embodiment, said chamber structure forming material is one or more selected from a group consisting of povidone, polyoxyethylene, copovidone and lactose; preferably povidone and copovidone; more preferably copovidone.

In another preferred embodiment, said chamber structure shape-modulating agent is acid or basic material or salts thereof; preferably, said acid or basic material or salts thereof is one or more selected from a group consisting of sodium phosphate, disodium hydrogen phosphate, sodium dihydrogen phosphate, sodium chloride, potassium chloride, sodium hydroxide, amino acid, citric acid and tartaric acid.

In another preferred embodiment, based on the total weight of the core, said core comprises: 1-60 wt% of drug(s), 5-90 wt% of chamber structure forming material(s), 1-50 wt% of chamber structure shape-modulating agent(s), 0.5-10 wt% of lubricating agent(s), and 1-60 wt% of pharmaceutical acceptable auxiliary material(s).

In another preferred embodiment, said coating film is permeable to water but not to drug active ingredients (except for the drug releasing pore).

In another preferred embodiment, said coating film contains coating material, optional pore forming agent and optional plasticizer.

In another preferred embodiment, based on the total weight of the coating film, said coating film comprises: 40-90 wt% of semi-permeable film coating material(s), 5-40 wt% of pore forming agent(s), and 0-20 wt% of plasticizer(s).

In another preferred embodiment,
based on the total weight of the core, said core comprises: 20-50 wt% of drug(s), 10-60wt% of chamber structure forming material(s), 5-40 wt% of chamber structure shape-modulating agent(s), 1-5 wt% of lubricating agent(s), and 5-40 wt% of pharmaceutical acceptable auxiliary material(s);
based on the total weight of the coating film, said coating film comprises: 60-80 wt% of semi-permeable coating material(s), 10-30 wt% of pore forming agent(s), and 0-15 wt% of plasticizer(s).

In another preferred embodiment, said chamber structure shape-modulating agent is an acid or basic material or salts thereof.

In another preferred embodiment, said acid or basic material or salts thereof is one or more selected from a group consisting of sodium phosphate, disodium hydrogen phosphate, sodium dihydrogen phosphate, sodium chloride, potassium chloride, sodium hydroxide, amino acid, citric acid and tartaric acid.

In another preferred embodiment, said lubricating agent is one or more selected from a group consisting of stearic acid, magnesium stearate, talc powder, sodium stearyl fumarate and paraffin.

In another preferred embodiment, said pharmaceutical acceptable auxiliary material (or carrier) includes one or more selected from a group consisting of binders, opacifier, filling agent, colorant, antioxidant and osmotic pressure promoting agent.

In another preferred embodiment, said semi-permeable film coating material is one ore more selected from a group consisting of cellulose acetate, cellulose acetate phthalate, ethyl cellulose, and acrylic resin and hydroxypropyl cellulose phthalate.

In another preferred embodiment, said pore forming agent is one or more selected from a group consisting of polyethylene glycol, povidone, urea and hydroxypropyl methylcellulose.

In another preferred embodiment, said drug is water soluble drug or water insoluble drug.

In another preferred embodiment, said water soluble drug includes, but not limited to: captopril, ribavirin, ketorolac tromethamine, levocarnitine, pentoxifylline, salvianolic acid, doxofylline, metformin, alfuzosin hydrochloride, ligustrazine phosphate, diltiazem hydrochloride, lamivudine or salbutamol sulfate; water insoluble drug includes but not limited to, ibuprofen, ketoprofen, azithromycin, roxithromycin, glipizide, felodipine, nimodipine, nisoldipine, nitrendipine, nifedipine, doxazosin mesylate, risperidone, famotidine, huperzine a, gliquidone, simvastatin, ambroxol hydrochloride, barnidipine, etodolac, acipimox, indapamide, azelnidipine, nimesulide, levetiracetam, tamsulosin hydrochloride, pitavastatin calcium, etodolic acid.

Preferably, said water insoluble drug includes glipizide, azithromycin, roxithromycin, ketoprofen, ibuprofen; said water soluble drug includes captopril.

The second aspect of the present invention, a method for designing a target preparation with desired drug releasing rate or releasing curve is provided, comprising the steps of:

(i) providing a drug osmotic pump preparation according to the first aspect of the invention, wherein said preparation comprises said drug as an active ingredient;

(ii) placing said drug osmotic pump preparation into water or aqueous solvent, and detecting the evolution situation of the micro chamber structures in said drug osmotic pump preparation;

(iii) calculating the releasing rate or releasing curve of the drug according to the detected evolution situation of the micro chamber structures;

(iv) selecting a drug osmotic pump preparation as a target preparation from the detected preparations, the releasing rate or releasing curve of which is close to or in accordance with desired releasing rate or releasing curve of the drug.

In another preferred embodiment, step (iv) further comprises: based on the detected releasing rate or releasing curve, selecting a preparation A, the releasing rate or releasing curve of which is close to that of the desired drug, modulating the formulation according to preparation A and repeating step (i), (ii) and (iii) for one or more times, thereby selecting a drug osmotic pump preparation as the target preparation from the detected preparations, the releasing rate or releasing curve of which is closer to or in accordance with desired drug releasing rate or releasing curve. In another preferred embodiment, the modulation of formulation includes modulating the composition ratio between chamber structure forming material and chamber structure shape-modulating agent.

In another preferred embodiment, the modulation of the formulation comprises:

when the detected releasing rate is faster than desired releasing rate, the amount of chamber structure shape-modulating agent is decreased or the ratio between said chamber structure forming material and chamber structure shape-modulating agent is increased;

when the detected releasing rate is slower than desired releasing rate, the amount of chamber structure shape-modulating agent is increased or the ratio between said chamber structure forming material and chamber structure shape-modulating agent is decreased.

In another preferred embodiment, microimaging is used for detection in step (ii), preferably synchrotron radiation light source microimaging.

In the third aspect of the invention, a preparation method for drug osmotic pump preparation according to the first aspect of the invention is provided, comprising the steps of: providing a core, wherein said core comprises drug(s) (or drug active ingredient(s)) and chamber structure forming material(s);

coating said core, to form a coating film covering said core;
punching said coating film, to form drug releasing pore on said coating film.

In another preferred embodiment, said core is prepared as follows:

(a) providing a mixture of chamber structure forming material(s), chamber structure shape-modulating agent(s) and/or pharmaceutical acceptable auxiliary material(s) or lubricating agent(s);
(b) tabletting or granulating the mixture of step (a) to form said core.

In another preferred embodiment, during said preparation of the core, firstly, a first mixture is formed by mixing the chamber structure forming material(s) with the chamber structure shape-modulating agent(s); then, the first mixture is mixed with drug(s), optional lubricating agent(s) and optional pharmaceutical acceptable auxiliary material(s), thereby obtaining the core.

The fourth aspect of the invention is to provide a drug osmotic pump preparation comprising core and coating film, wherein
based on the total weight of the core, said core comprises: 1-60 wt% of drug(s), 5-90 wt% of chamber structure forming material(s), 1-50 wt% of chamber structure shape-modulating agent(s), 0.5-10 wt% of lubricating agent(s), and 1-60 wt% of pharmaceutical acceptable auxiliary material(s);
based on the total weight of the coating film, said coating film comprises: 40-90 wt% of semi-permeable film coating material(s), 5-40 wt% of pore forming agent(s), and 0-20 wt% of plasticizer(s).

In another preferred embodiment, said drug osmotic pump preparation comprises:

a core comprising drug(s) (or drug active ingredient(s)) and chamber structure forming material(s); and
a coating film for covering the core with drug releasing pore(s) set on said coating film;
wherein, when the drug osmotic pump preparation is used for drug release in water or aqueous medium, micro chamber structures are thus formed in the coating film by the chamber structure forming material(s) and total volume of the micro chamber structure Vb increases with releasing time, and releasing rate R of drug active ingredients (or releasing amount A) is directly proportional to Vb.

In another preferred embodiment, based on the total weight of the core, said core comprises: 20-50 wt% of drug(s), 10-60wt of chamber structure forming material(s), 5-40 wt% of chamber structure shape-modulating agent(s), 1-5 wt% of lubricating agent(s), and 5-40 wt% of pharmaceutical acceptable auxiliary material(s);
based on the total weight of the coating film, said coating film comprises: 60-80 wt% of semi-permeable film coating material(s), 10-30 wt% of pore forming agent(s), and 0-15 wt% of plasticizer(s).

In another preferred embodiment, said chamber structure forming material is one or more selected from a group consisting of povidone, polyoxyethylene, copovidone and lactose; preferably povidone and copovidone; more preferably copovidone.

In another preferred embodiment, said chamber structure shape-modulating agent is acid or basic material or salts thereof.

In another preferred embodiment, said pharmaceutical acceptable auxiliary material or carrier includes one or more selected from a group consisting of binders, opacifier, filling agent, colorant, antioxidant and osmotic pressure promoting agent.

It should be understood that in the present invention, the technical features specifically described above and below (such as the Examples) can be combined with each other, thereby constituting a new or preferred technical solution which needs not be described one by one.

Descriptions of Figures

Figure 1 shows a tomography image of internal micro vesicular structures formed in the osmotic pump tablet after 1.0 hr dissolution in Example 1;
Figure 2 shows the 3-D distribution of the internal micro vesicular structures of the osmotic pump tablet after 1.0 hr dissolution in Example 1;
Figure 3 shows the 3-D structure of solid residues from the osmotic pump tablet after 1.0 hr dissolution in Example 1;
Figure 4 shows a tomography image of the internal micro vesicular structures formed in the osmotic pump tablet after 1.0 hr dissolution in Example 2;
Figure 5 shows the 3-D distribution of the internal micro vesicular structures of the osmotic pump tablet after 1.0 hr dissolution in Example 2;

Figure 6 shows the 3-D distribution of internal micro vesicular structures-modulating agent of the osmotic pump tablet after 1.0 hr dissolution in Example 2;

Figure 7 shows 3-D distribution of the space-associated relation between internal micro vesicular structures and modulating agent in the osmotic pump tablet after 1.0 hr dissolution in Example 2;

Figure 8 shows the 3-D structure of solid residues from the osmotic pump tablet after 1.0 hr dissolution in Example 2;

Figure 9 shows a tomography image of the internal micro vesicular structures formed in the osmotic pump tablet after 1.0 hr dissolution in Example 3;

Figure 10 shows the 3-D distribution of the internal micro vesicular structures of the osmotic pump tablet after 1.0 hr dissolution in Example 3;

Figure 11 shows the 3-D structure of solid residues from the osmotic pump tablet after 1.0 hr dissolution in Example 3;

Figure 12 shows a tomography image of the internal micro vesicular structures formed in the osmotic pump tablet after 2.0 hr dissolution in Example 3;

Figure 13 shows the 3-D distribution of the internal micro vesicular structures of the osmotic pump tablet after 2.0 hr dissolution in Example 3;

Figure 14 shows the 3-D structure of solid residues from the osmotic pump tablet after 2.0 hr dissolution in Example 3;

Figure 15 shows a tomography image of the internal micro vesicular structures formed in the osmotic pump tablet after 1.0 hr dissolution in Example 4;

Figure 16 shows the 3-D distribution of the internal micro vesicular structures of the osmotic pump tablet after 1.0 hr dissolution in Example 4;

Figure 17 shows the 3-D distribution of internal micro vesicular structures-modulating agent of the osmotic pump tablet after 1.0 hr dissolution in Example 4;

Figure 18 shows the 3-D distribution of the space-associated relation between internal micro vesicular structures and modulating agent in the osmotic pump tablet after 1.0 hr dissolution in Example 4;

Figure 19 shows the 3-D structure of solid residues from the osmotic pump tablet after 1.0 hr dissolution in Example 4;

Figure 20 shows a tomography image of the internal micro vesicular structures formed in the osmotic pump tablet after 2.0 hr dissolution in Example 4;

Figure 21 shows the migration rules of the internal micro vesicular structures in Example 4;

Figure 22 shows the time series tomography image of the internal micro vesicular structures in Example 4;

Figure 23 shows the 3-D distribution of the internal micro vesicular structures of the osmotic pump tablet in Example 4;

Figure 24 shows the 3-D distribution of the internal micro vesicular structure modulating agent in the osmotic pump tablet in Example 4;

Figure 25 shows the 3-D distribution of the space-associated relation between internal micro vesicular structures and modulating agent in the osmotic pump tablet in Example 4;

Figure 26 shows the 3-D structure of solid residues from the osmotic pump tablet in Example 4;

Figure 27 shows the *in vitro* time-accumulated releasing curve of ketoprofen osmotic pump tablet in Example 4;

Figure 28 shows the correlation between the accumulated releasing rate and the total volume of internal micro vesicular structures in Example 4;

Figure 29 shows the evolution situation over time of the space coordinates of the internal micro vesicular in Example 4;

Figure 30 shows the evolution situation over time of the volume distribution of the internal micro vesicular in Example 4;

Figure 31 shows a tomography image of the internal micro vesicular structures formed in the osmotic pump tablet after 1.0 hr dissolution in Example 5;

Figure 32 shows the 3-D distribution of the internal micro vesicular structures of the osmotic pump tablet after 1.0 hr dissolution in Example 5;

Figure 33 shows the 3-D distribution of internal micro vesicular structures-modulating agent of the osmotic pump tablet after 1.0 hr dissolution in Example 5;

Figure 34 shows the 3-D distribution of the space-associated relation between internal micro vesicular structures and modulating agent in the osmotic pump tablet after 1.0 hr dissolution in Example 5;

Figure 35 shows the 3-D structure of solid residues from the osmotic pump tablet after 1.0 hr dissolution in Example 5;

Figure 36 shows a tomography image of the internal micro vesicular structures formed in the osmotic pump tablet after 1.0 hr dissolution in Example 6;

Figure 37 shows the 3-D distribution of the internal micro vesicular structures of the osmotic pump tablet after 1.0 hr dissolution in Example 6;

Figure 38 shows the 3-D distribution of the internal micro vesicular structure modulating agent of the osmotic pump tablet after 1.0 hr dissolution in Example 6;

Figure 39 shows the 3-D distribution of the space-associated relation between internal micro vesicular structures and modulating agent in the osmotic pump tablet after 1.0 hr dissolution in Example 6;

Figure 40 shows the 3-D structure of solid residues from the osmotic pump tablet after 1.0 hr dissolution in Example 6;

Figure 41 shows a tomography image of the internal micro vesicular structures formed in the osmotic pump tablet after 1.0 hr dissolution in Example 7;

Figure 42 shows the 3-D distribution of the internal micro vesicular structures of the osmotic pump tablet after 1.0 hr dissolution in Example 7;

Figure 43 shows the 3-D distribution of the internal micro vesicular structure modulating agent of the osmotic pump tablet after 1.0 hr dissolution in Example 7;

Figure 44 shows the 3-D distribution of the space-associated relation between internal micro vesicular structures and modulating agent in the osmotic pump tablet after 1.0 hr dissolution in Example 7;

Figure 45 shows the 3-D structure of solid residues from the osmotic pump tablet after 1.0 hr dissolution in Example 7;

Figure 46 shows a tomography image of the internal micro vesicular structures formed in the osmotic pump tablet after 2.0 hr dissolution in Example 7;

Figure 47 shows the 3-D distribution of the internal micro vesicular structures of the osmotic pump tablet after 2.0 hr dissolution in Example 7;

Figure 48 shows the 3-D distribution of the internal micro vesicular structure modulating agent of the osmotic pump tablet after 2.0 hr dissolution in Example 7;

Figure 49 shows the 3-D distribution of the space-associated relation between internal micro vesicular structures and modulating agent in the osmotic pump tablet after 2.0 hr dissolution in Example 7;

Figure 50 shows the 3-D structure of solid residues from the osmotic pump tablet after 2.0 hr dissolution in Example 7;

Figure 51 shows a tomography image of the internal micro vesicular structures formed in the osmotic pump tablet after 1.0 hr dissolution in Example 8;

Figure 52 shows the 3-D distribution of the internal micro vesicular structures of the osmotic pump tablet after 1.0 hr dissolution in Example 8;

Figure 53 shows the 3-D distribution of the internal micro vesicular structure modulating agent of the osmotic pump tablet after 1.0 hr dissolution in Example 8;

Figure 54 shows the 3-D distribution of the space-associated relation between internal micro vesicular structures and modulating agent in the osmotic pump tablet after 1.0 hr dissolution in Example 8;

Figure 55 shows the 3-D structure of solid residues from the osmotic pump tablet after 1.0 hr dissolution in Example 8;

Figure 56 shows a tomography image of the internal micro vesicular structures formed in the osmotic pump tablet after 2.0 hr dissolution in Example 8;

Figure 57 shows the 3-D distribution of the internal micro vesicular structures of the osmotic pump tablet after 2.0 hr dissolution in Example 8;

Figure 58 shows the 3-D distribution of the internal micro vesicular structure modulating agent of the osmotic pump tablet after 1.0 hr dissolution in Example 8;

Figure 59 shows the 3-D distribution of the space-associated relation between internal micro vesicular structures and modulating agent in the osmotic pump tablet after 2.0 hr dissolution in Example 8;

Figure 60 shows the 3-D structure of solid residues from the osmotic pump tablet after 2.0 hr dissolution in Example 8;

Figure 61 shows a tomography image of the internal micro vesicular structures formed in the osmotic pump tablet after 1.0 hr dissolution in Example 9;

Figure 62 shows the *in vitro* time-accumulated releasing curve of azithromycin osmotic pump tablet in Example 10;

Figure 63 shows a tomography image of the internal micro vesicular structures formed in azithromycin osmotic pump after 1.0 hr dissolution in Example 10;

Figure 64 shows the *in vitro* time-accumulated releasing curve of captopril osmotic pump tablet in Example 11;

Figure 65 shows a tomography image of the internal micro vesicular structures formed in captopril osmotic pump after 1.0 hr dissolution in Example 10;

## Detailed Embodiments

Upon intensive and extensive studies, the inventors unexpectedly discovered that adding high portion of chamber structure forming material into the drug osmotic pump preparation allows huge amounts of micro chamber structures formed within the coating film with total volumes of micro chamber structures increasing over time, when drug osmotic pump preparation releases drugs in aqueous medium. Different modes of controlled release can be achieved effectively by the osmotic pump of the present invention through the evolution mechanism of the internal structures of the core. Upon modulating the ratio between chamber structures modulating agent and chamber structure forming material, a ratio matching the releasing characteristics of the desired drug can be designed and screened, thereby designing different modes of controlled drug releasing according to different drug characteristics. Based on the above works, the present invention is completed.

**Terms**

As used herein, term "chamber structure forming material" or "micro chamber structure forming material" can be used interchangeably, referring to a material(s) capable of forming micro bubble-shaped, porous and vesicular chamber-shaped structures inside of the coating film when contacting with water.

Generally, the chamber structure forming material is an artificially synthesized high molecular material or a nature high molecular material. One or more selected from these materials can form micro chambers inside of the coating film of osmotic pump preparation, wherein these materials include, but not limited to one or more selected from a group consisting of povidone, polyoxyethylene, copovidone and lactose. Copovidone, polyoxyethylene-copovidone combination, povidone-copovidone combination or lactose-copovidone is preferred.

Based on the total weight of the core, the content of the chamber structure forming material is not specifically limited, and can be any amount that allows the core to form micro chamber structures and release drug active ingredients after the osmotic pump contacts with aqueous medium. Typically, the amount is 15-99.8 wt%, preferably is 20-95 wt%, and more preferably is 30-90 wt%.

As used herein, term "chamber structure shape-modulating agent", "chamber structure modulating agent" and "micro chamber modulating agent" can be used interchangeably, referring to a agent or material used for modulating or affecting the forming process (such as forming rate, size, profile) of the micro chambers. Since the profile or the shape of micro chamber structures varies with the modulation by using different modulating agents, these materials can be collectively named as "chamber structure shape-modulating agent". Generally, based on the total weight of the core, the amount of the chamber structure shape-modulating agent is 0-50 wt%, preferably 1-50 wt %, more preferably 5-40 wt%.

As used herein, term "drug" or "drug active ingredient" can be used interchangeably, referring to the drug active ingredient mainly exerting therapeutic effect in human.

As used herein, term "drug release pore", "micro pore" or "drug-releasing pore" can be use interchangeably, referring to the pore located on the coating film for drug releasing. Generally, the located drug releasing pore includes a drug releasing pore preformed on the film by laser or a punching machine, or a releasing pore formed by an agent, such as pore forming agent, capable of forming drug releasing pore in the coating film. Pore forming agent is preferred

Diameter of drug releasing pore is not specifically limited as long as it allows the drug inside of the core releasing from the coating film. Typically, diameter of drug releasing pore is 0.001-3 mm, preferably is 0.01-2 mm, more preferably is 0.05-1.2 mm, most preferably is 0.2-1 mm.

As used herein, term "drug releasing rate" or "drug releasing amount" refers to drug releasing extent and amount of the osmotic pump preparation according to the present invention. Generally, it is represented by percentage of drug releasing weight to the drug weight in the core.

**Structure of Osmotic Pump Preparation**

The structure of the osmotic pump preparation according to the present invention from the inside to the outside is a coating film (and the drug releasing pore on the film) and a core. A osmotic pump preparation without power layer structure is preferred.

Wherein, the core of osmotic pump preparation according to the present invention contains drug active ingredient(s), chamber structure forming material(s) and optional chamber structure shape-modulating agent(s), lubricating agent(s), antioxidant(s), osmotic pressure promoting agent(s), binder(s), filling agent(s) or other pharmaceutical acceptable auxiliary material(s). When the core contacts with water or aqueous medium, micro chamber structures in liquid state are formed by chamber structure forming material and active ingredients in the core etc, and the micro chamber structures fuse and release (drug) slowly through filming coat over time.

Drugs which can be used for the osmotic pump preparation according to the present invention include water soluble drugs or water insoluble drugs.

The water soluble drug which can be used in the present invention includes but not limited to captopril, ribavirin, ketorolac tromethamine, levocarnitine, pentoxifylline, salvianolic acid, doxofylline, metformin, alfuzosin hydrochloride, ligustrazine phosphate, diltiazem hydrochloride, lamivudine or salbutamol sulfate.

The water insoluble drug which can be used in the present invention includes but not limited to ibuprofen, ketoprofen, azithromycin, roxithromycin, glipizide, felodipine, nimodipine, nisoldipine, nitrendipine, nifedipine, doxazosin mesylate, risperidone, famotidine, huperzine a, gliquidone, simvastatin, ambroxol hydrochloride, barnidipine, etodolac, acipimox, indapamide, azelnidipine, nimesulide, levetiracetam, tamsulosin hydrochloride, pitavastatin calcium, etodolic acid.

Preferably, said water insoluble drug includes glipizide, azithromycin, roxithromycin, ketoprofen, or ibuprofen.

The chamber structure forming material which can be used in the present invention includes but not limited to one or more selected from a group consisting of povidone, polyoxyethylene, copovidone or lactose. Povidone or copovidone is preferred.

The chamber structure shape-modulating agent which can be used in the present invention is acid or basic material or salts thereof. Preferably, said acid or basic material or salts thereof is one or more selected from a group consisting of sodium phosphate, disodium hydrogen phosphate, sodium dihydrogen phosphate, sodium chloride, potassium chloride, sodium hydroxide, amino acid, citric acid and tartaric acid. Sodium chloride, sodium phosphate, or citric acid is preferred.

The effect on the shape of the chamber structure varies with different chamber structure modulating agents. For example, when sodium phosphate is used, the micro chamber structure formed is basically a regular sphere. When other chamber structure shape-modulating agents, such as sodium chloride are used, the formed micro chamber structures are usually irregular.

The lubricating agent, antioxidant, opacifier, osmotic pressure promoting agent, binder, filling agent or other pharmaceutical acceptable auxiliary materials which can be used in the present invention need not be specifically limited. It can be any agent suitable for osmotic pump preparation and well known to those skilled in the art. Generally, these pharmaceutical acceptable auxiliary materials are not toxic themselves, do not react with the active ingredients in the core or chamber structure forming materials, and can be safely used in the core to facilitate the administration of the osmotic pump preparation according to the present invention.

The binder which can be used in the present invention is not specifically limited. Preferably, it includes one or more selected from a group consisting of starch slurry, povidone, hydroxypropyl cellulose, hydroxypropyl methyl cellulose, methyl cellulose, gelatin and polyethylene glycol; preferably, said opacifier is one or more of titanium oxide, talc powder and silicon dioxide; preferably, said filling agent is one or more of lactose, mannitol, microcrystalline cellulose, starch, dextrin, and calcium carbonate; said colorant is one or more selected from red iron oxide and yellow iron oxide; preferably, said osmotic pressure promoting agent is one or more of sodium chloride, potassium chloride and soluble saccharides. The lubricating agent which can be used in the core of the present invention is preferably one or more from stearic acid, magnesium stearate, talcum powder, sodium stearyl fumarate and paraffin.

Generally, there is drug releasing pore formed by laser or punching machine located on the coating film of the osmotic pump preparation according to the present invention. Wherein, there can be one or more drug releasing pore(s) located on one or more sides of the preparation with a size between 0.01-3 mm, preferably 0.1-2mm, more preferably 0.5-1.2mm. Furthermore, a coating film without punched pore is also acceptable since the drug releasing pore can be formed by pore forming agent or plasticizer added into the coating film. The coating film of the osmotic pump preparation according to the present invention is semi-permeable coating material, which is permeable to water molecules or aqueous solvent while impermeable to the ingredients in the core.

The semi-permeable material which can be used in the present invention is one or more selected from a group consisting of cellulose acetate, cellulose acetate phthalate, ethyl cellulose, acrylic resin and hydroxypropyl cellulose phthalate.

The pore forming agent which can be used in the coating film of the osmotic pump preparation according to the present invention is one or more selected from a group consisting of polyethylene glycol, povidone, urea, and hydroxypropyl methylcellulose. The main effect of said pore forming agent is to modulate the permeability of the semi-permeable film, which is one or more selected from a group consisting of polyethylene glycol (polyethylene glycol 200, polyethylene glycol 1450, polyethylene glycol 1500, polyethylene glycol 4000, polyethylene glycol 6000), povidone, urea, and hydroxypropyl methylcellulose; preferably, polyethylene glycol 4000. Certainly, said coating film also includes solvent for coating solution, and said solvent for coating solution is one or more selected from a group consisting of ethanol absolute, methanol, acetone, and water; preferably, acetone.

The coating film of the osmotic pump preparation according to the present invention can further comprise plasticizer, and said plasticizer includes triethyl citrate, polyethylene glycol, diethyl phthalate, dibutyl sebacate, glycerol triacetate, castor oil, phthalic acid ester; preferably, phthalic acid ester.

The coating film of the osmotic pump preparation according to the present invention further comprises colorant or other pharmaceutical acceptable auxiliary materials.

The content of each component in the coating film according to present invention is not specifically limited. Any content suitable for osmotic pump preparation is acceptable. Generally, based on the total weight of the coating film, said coating film contains: 40-90 wt%, preferably 60-80 wt% of semi permeable coating material(s); optional 5-40 wt%, preferably 10-30 wt% of pore forming agent(s); or optional 0-20 wt%, preferably 0-15 wt% of plasticizer(s).

The macrostructure of the osmotic pump preparation according to the present invention can be generally prepared into single layer tablet or multiple layers tablet, including single layer osmotic pump and multiple layers osmotic pump with a volume of 0.05-5000 cm$^3$, preferably 10-2000 cm$^3$ and a weight of 5-5000 mg, preferably 10-2000 mg.

**Core and Preparation Thereof**

The present invention provides a novel drug osmotic pump preparation comprising a core and a coating film, and based on the total weight of the core, said core comprises:

0.2-99%, preferably 1-60 wt%, preferably 20-50 wt% of drug(s);
15-99.8 wt%, preferably 20-95wt % , more preferably 30-90 wt% of chamber structure forming material(s);
or optional 0-50wt%, preferably 1-50wt%, more preferably 5-40 wt% of chamber structure shape-modulating agent(s);
or optional 0-10 wt%, preferably 0.5-10 wt%; more preferably 1-5 wt% of lubricating agent(s);
or optional 0-60 wt%, preferably 1-60 wt%, more preferably 5-40 wt% of pharmaceutical acceptable auxiliary material(s).

The preparation method for the core of the osmotic pump according to the present invention is not specifically limited. Any conventional method (such as a direct mixing) can be used for the preparation. Preferably, drug(s) and chamber structure forming material(s) can be mixed homogeneously at a desired ratio to form the first mixture, and optional chamber structure shape-modulating agent(s), lubricating agent(s), or other pharmaceutical acceptable auxiliary material(s) can be added in random order into the first mixture to form the second mixture. Then the second mixture is granulated or tableted without granulation to form the core of the present invention.

**Micro Chamber Structure**

As used herein, term "micro chamber structure" or "chamber structure" refers to the spherical, bubble-shaped, porous or vesicular micro structure inside the coating film formed after osmotic pump preparation according to the present invention contacts with water. Preferably, said micro chamber structure and chamber structure are liquid structure.
Wherein, "micro chamber structure" can be determined by microimaging, preferably by synchrotron radiation light source microimaging, and chamber structure with a minimum diameter as low as about 5-10 $\mu$m can be observed. Micro chamber structures will fuse and merge with the release of the drugs over time to form micro chamber structures or further chamber structures with lager volume. Generally, although a "micro chamber structure" can be used when the volume is over 10mm$^3$, the fused chamber structures with larger volume are named as chamber structures for convenience.
The micro chamber structures according to the present invention is characterized in the dynamic change of internal structures of micro chamber during the releasing process and the correlation between three-dimensional characteristic parameters such as total volume of vesicular structure involving structural changes and the releasing rate of drugs. Drug release is controlled by modulating the profile and three-dimensional characteristics of the micro chamber structures. The formed micro chamber structures include regular sphere, irregular sphere, the transition and mixture of regular and irregular shape, and the size evolution of the micro chambers.
Generally, the total volume of the micro chamber structures is represented by Vb, and typically, Vb refers to the volume of micro chamber structure and the volume of chamber structure formed by two or more micro chamber structures. Wherein, the ratio between the maximum value of Vb, Vbmax and the core volume Va is 90-100: 100, preferably is 95-100: 100. Furthermore, the internal volume of the coating film Vc can be generally deemed as identical with Va, which is identical with a profile volume of osmotic pump shaped with coating film, Vm. Therefore, Vb=Va=Vc=Vm, when Vb reaches to Vbmax. When the total amount of micro chamber structures keeps unchanged or declines, the average volume of said micro chamber structures gradually increases over time.

The Principle of the Positive Correlation between Micro Chamber Structure and Drug Releasing

Micro bubble-shaped structures are formed inside the osmotic pump preparation according to the present invention when it contacts with water. The total volume of bubble-shaped , Vb gradually increases from 0; while the profile volume (Vm) of the osmotic pump formed by the outer film of the tablet may slightly increase when contacts with water, but essentially keeps unchanged during the releasing process. Thus,

$$\lim_{t=t_{\max}} V_b = V_m \qquad\qquad (1)$$

If the volume of solid inside the core equals to Vd, when the solid inside the core contacts with water under an atmospheric pressure (that is, it is not in the osmotic pump with thorough hydration) at the "concentration" of semi-solid which is excreted through the pore of osmotic pump. Then set:

$$a = \frac{V_d}{V_m} \qquad (2)$$

Obviously, at t moment, the percentage of the solid excreted from the tablet to the total amount of the tablet essentially corresponds to the releasing rate R, which is:

$$R \approx \frac{a}{V_d} V_b \qquad (3)$$

Since both of a and Vd refer to a constant k, the releasing rate of this type of osmotic pump is directly proportional to the volume of the bubble-shaped structures, which can be simplified that drug releasing rate is directly proportional to the volume of the internal-formed vesicular structures:

$$R = kV_b \qquad (4)$$

**Designing Method for A Target Preparation and Modulation Method for Micro Chamber Structures**

According to the present invention, the formulation of a desired drug preparation can be determined by comparing different drug release with the drug release obtained from the osmotic pump preparation of the present invention. Specifically, a method for designing a target preparation with desired drug releasing rate or releasing curve comprises:

(i) providing a drug osmotic pump preparation according to the present invention, wherein said preparation comprises said drug as active ingredient;
(ii) placing said drug osmotic pump preparation into water or aqueous solvent, and detecting the evolution situation of the micro chamber structures in said drug osmotic pump preparation;
(iii) calculating the releasing rate or releasing curve of the drug according to the detected evolution situation of the micro chamber structures;
(iv) selecting a drug osmotic pump preparation as a target preparation from the detected preparations, the releasing rate or releasing curve of which is close to or in accordance with desired drug releasing rate or releasing curve.

Preferably, step (iv) further comprises: based on the detected releasing rate or releasing curve, selecting a preparation A, the releasing rate or releasing curve of which is close to that of the desired drug, modulating the formulation according to preparation A and repeating step (i), (ii) and (iii) for one or more times, thereby selecting a drug osmotic pump preparation as the target preparation from the detected preparations, the releasing rate or releasing curve of which is closer to or in accordance with desired drug releasing rate or releasing curve. Preferably, the modulation of formulation includes modulating the composition ratio between chamber structure forming material and chamber structure shape-modulating agent so as to modulate the profile and the size of the micro chamber as well as the evolution rate between the profile and the size of the micro chamber.

Said chamber structure shape-modulating agent is used to modulate the size and profile evolution of the bubble-shaped micro structure, and can be acid/basic material or salts thereof with different solubility, including inorganic acid or salts thereof and inorganic base, including one or more selected from a group consisting of sodium phosphate, disodium hydrogen phosphate, sodium dihydrogen phosphate, sodium chloride, potassium chloride, sodium hydroxide and sodium phosphate or disodium hydrogen is preferred; and organic acid or salts thereof, comprising one or more selected from a group consisting of amino acid, citric acid and tartaric acid and citric acid is preferred.

The method for modulating the size of micro chamber includes modulating the amount of chamber structure shape-modulating agent (1-50 wt%) as well as the particle size (10-1000 $\mu$m) and the particle size of the core powder (10-100 $\mu$m).

The method for modulating the evolution rate between the profile and the size of micro chambers includes modulating the combination of micro chamber forming material and micro chamber modulating agent, involved viscosity and hydration rate. The detailed evolution rules include that the number of chamber can be increased or decreased by adding chamber structure shape-modulating agent; the faster the hydration rate of chamber forming material is, the faster the chamber evolves; the higher the micro environment viscosity of the entire core is, the slower the chamber evolves.

The particle fluidity and homogeneity of drug preparation content can be improved by adding lubricating agent; and said lubricating agent includes, but not limited to one or more selected from a group consisting of stearic acid, magnesium stearate, talc powder, sodium stearyl fumarate and paraffin, and the mixture thereof.

A preferred method for adjusting the formulation comprises:

when the detected releasing rate is faster than desired releasing rate, decreasing the amount of chamber structure shape-modulating agent or increasing the ratio between said chamber structure forming material and chamber structure shape-modulating agent;

when the detected releasing rate is slower than desired releasing rate, increasing the amount of chamber structure shape-modulating agent or decreasing the ratio between said chamber structure forming material and chamber structure shape-modulating agent.

**Sample Treatment and Determination of Internal Structure of the Preparation**

The determination method of the bubble-shaped, porous or vesicular micro chamber structure according to the present invention includes solidifying the tablet sample used in releasing determination, then conducting microimaging, typically synchrotron radiation light source X-ray micro CT for 3D reconstruction, or conducting microsection, superimposing images of multiple slices and then conducting 3D reconstruction, but not limited to the above methods. Preferably, the sample treatment and determination of internal structure of the preparation according to the present invention includes the following steps:

(1) Sample treatment. The osmotic pump tablet is removed at a desired testing time point during the dissolving test process, and dried under the condition that the internal structure of the sample keeps unchanged, which includes placing it in the sealed drier filled with drying agent under constant temperature and humidity or lyophilization.

(2) Synchrotron radiation light source X-ray micro CT scanning. According to the sample formulation and the components thereof, testing parameters such as X-ray energy, X-ray flux, exposure time and field parameters (energy 13 kev, exposure time, 2 s, CDD 3.7 $\mu$m) are optimized, a proper CCD camera is selected, and two-dimensional perspective projection images of the samples, background and black field are collected. A proper rotational angular velocities Vel and an exposure time are set, and photos are automatically taken by the system at every rotation to a certain angle (0.25° angle interval, 720 projections were collected totally). 2-D images from 180° view are collected for synchrotron radiation light source X-ray micro CT 3-D scanning.

(3) 3-D reconstruction. Firstly, the collected projection images with different angle are subjected to noise reduction and background correction for improving definition and resolution of the images; CT images are subjected to position correction and 3-D reconstruction based on back projection algorithms; upon reconstruction, slicing parameters are set to section the reconstructed result for obtaining the slice tomography images of the sample in its horizontal direction, the tomography images are exported and screened.

The Beneficials Effect of the Present Invention:

1. The drug osmotic pump preparation according to the present invention allows the control of drug releasing rate by modulating the dynamic profile, size and evolution speed of micro chamber structures.

2. The drug osmotic pump preparation according to the present invention is suitable for the controlled releasing of water soluble drugs and water insoluble drugs, typically of drugs with low solubility or pH-dependent drugs. Said osmotic pump preparation can release drugs thoroughly at a controllable releasing rate.

3. Compare with the conventional osmotic pump preparations which control drug releasing through macrostructures, the osmotic pump preparation structure provided by the present invention has good controllability and feasibility.

The present invention will be illustrated in the following referring to the specific examples. These examples are only intended to illustrate the invention, but not to limit the scope of the invention. For the experimental methods in the following examples the specific conditions of which are not specifically indicated, they are performed under routine conditions or manufacturer's instruction. All the percentages or fractions refer to weight percentage and weight fraction, unless stated otherwise.

**Example 1:**

Copovidone sifted through a 100-mesh sifter was tableted directly. The tablet core was coated by cellulose acetate-PEG 4000 (7:1) acetone solution until the weight increasing of the core reached 4%; a pore with a diameter of 0.8 mm on one side of the coating tablet was punched by laser. Oar method was used and 900 mL of degassing distilled water was used as releasing medium at a rotate speed of 75 RPM. Releasing rates at different times were determined. Tablets at different times were removed, sealed in a dryer filled with drying agent under ambient temperature and left for 48 hours. 2-D images from 180°view were collected for SR-$\mu$CT 3-D scanning. The CT images were subjected to position correction and then 3-D reconstruction based on back projection algorithms. Upon reconstruction, the slicing parameters were set to section the reconstructed result for obtaining the slice tomography images of the sample in its horizontal direction, and the slice tomography images were exported and screened.

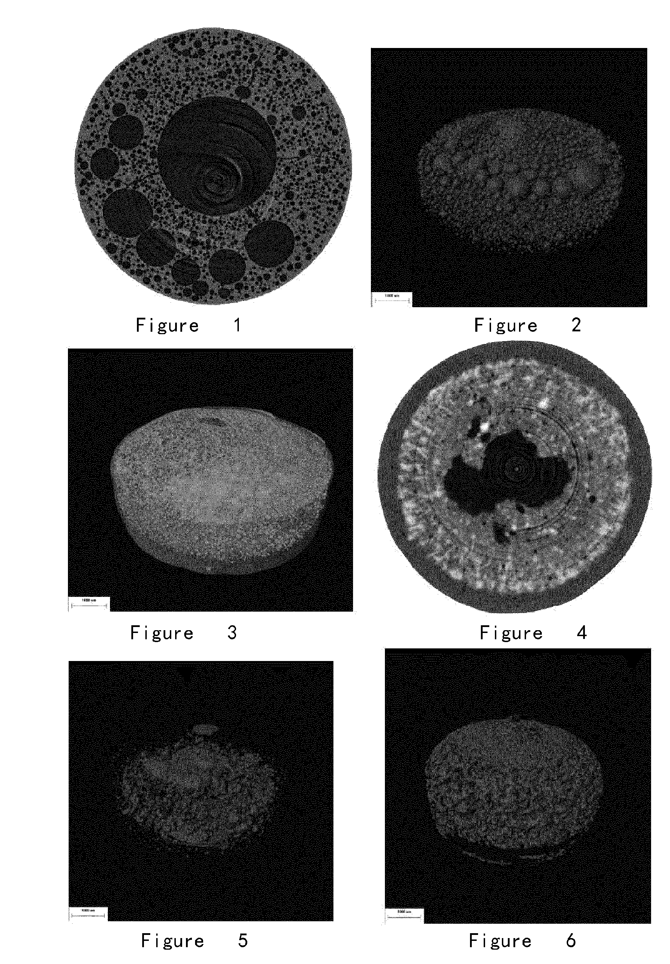

Result: 3-D tomography scanning images show that the obtained osmotic pump possesses micro bubble-shaped structures, wherein, the micro chamber structures formed after placed in a releasing medium for 1.0 hr (Figures 1, 2 and 3) show that the internal micro chamber structures have higher sphericity and copovidone is a preferable micro bubble-shaped structure forming material. Materials such as artificially synthesized high molecular materials or nature high molecular materials are used to form micro bubble-shaped, vesicular chamber structure with different 3-D profile, size, evolution mode and speed depending on their solubility, hydration rate, and system viscosity after hydration.

**Example 2:**

(1) Core formulation:

| components | mg / tablet |
| --- | --- |
| copovidone | 57.8 |
| sodium phosphate | 11 |
| magnesium stearate | 1.2 |

(2) Formulation for coating solution of the semi-permeable film:

| components | amount |
| --- | --- |
| cellulose acetate | 28 g |
| PEG 4000 | 4 g |
| acetone | 2000 mL |

(3) Preparation process: Sodium phosphate and copovidone sifted through a 100-mesh sifter were weighed and then mixed to homogeneous. Then magnesium stearate was added, mixed to homogeneous, and tableted. The core was coated by cellulose acetate-PEG 4000 acetone solution until the weight increasing of the core reached 4%; a pore with a diameter of 0.8 mm on one side of the coating film was punched by laser.

(4) Determination method: Oar method was used and 900 mL of degassing distilled water was used as releasing medium at a rotate speed of 75 RPM. Releasing rates at different times were determined. Osmotic pump tablets at different times were removed, sealed in a dryer filled with drying agent under ambient temperature and left for 48 hours. 2-D images from 180°view were collected for SR-$\mu$CT 3-D scanning. The CT images were subjected to position correction and then to 3-D reconstruction based on back projection algorithms. Upon reconstruction, the slicing parameters were set to section the reconstructed result for obtaining the slice tomography images of the sample in its horizontal direction, and the tomography images were exported and screened.

(5) Result: 3-D tomography scanning images show that the obtained osmotic pump possesses micro bubble-shaped structure, wherein, the micro chamber structures formed after placed in a releasing medium for 1.0 hr (Figures 4, 5, 6, 7 and 8) show that the internal micro chamber structures distribute radially towards the core center, and the volume increases with the chambers closer to the core center.

(6) Conclusion: Sodium phosphate can be used as micro bubble-shaped structure modulating agent to modulate the profile and evolution mode and speed of the micro bubble-shaped, vesicular chamber structures.

**Example 3:**

(1) Core formulation:

| components | mg / tablet |
|---|---|
| copovidone | 57.8 |
| ketoprofen | 50 |
| magnesium stearate | 1.2 |

(2) Formulation for coating solution of the semi-permeable film:

| components | amount |
|---|---|
| cellulose acetate | 28 g |
| PEG 4000 | 4 g |
| acetone | 2000 mL |

(3) Preparation process: Ditto to Example 2 except that ketoprofen and copovidone were mixed to homogenous and then the amount as listed in the formulation of magnesium stearate was added.

(4) Determination method: Ditto to Example 2

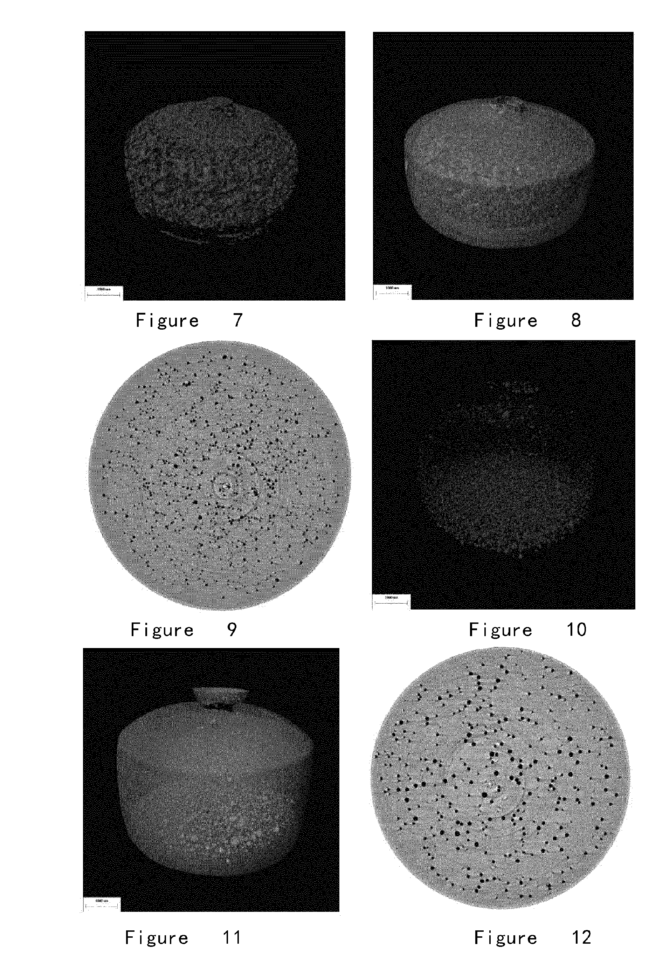

(5) Result: The internal chamber structures of the osmotic pump tablet obtained according to this formulation are regularly spherical. The micro chamber structures formed in osmotic pump tablet after 1.0-hour dissolution (Figure 9, 10 and 11) and the micro chamber structures formed in osmotic pump tablet after 2.0-hour dissolution (Figures 12, 13 and 14) show that the obtained micro spherical chamber structures are regular, uniformed and homogenously distributed. The amount of the chambers decreased and the diameter slightly increased due to the chamber fusion with the increasing time of hydration.

(6) Conclusion: As a insoluble drug with high doses, ketoprofen can remarkably promote the formation and stabilization of micro bubble-shaped structures with high sphericity and slow down the chamber evolution rate to some extent by increasing the system viscosity and decreasing hydration rate.

**Example 4:**

(1) Core formulation:

| components | mg / tablet |
|---|---|
| copovidone | 58 |
| ketoprofen | 50 |
| sodium phosphate | 10.8 |
| magnesium stearate | 1.2 |

(2) Formulation for coating solution of the semi-permeable film:

| components | amount |
|---|---|
| cellulose acetate | 28g |
| PEG4000 | 4g |
| acetone | 2000mL |

(3) Preparation process: Ditto to Example 2 except that ketoprofen and copovidone were mixed to homogenous, then sodium phosphate was added and mixed to homogenous, and finally, magnesium stearate was added.

(4) Determination method: Ditto to Example 2.

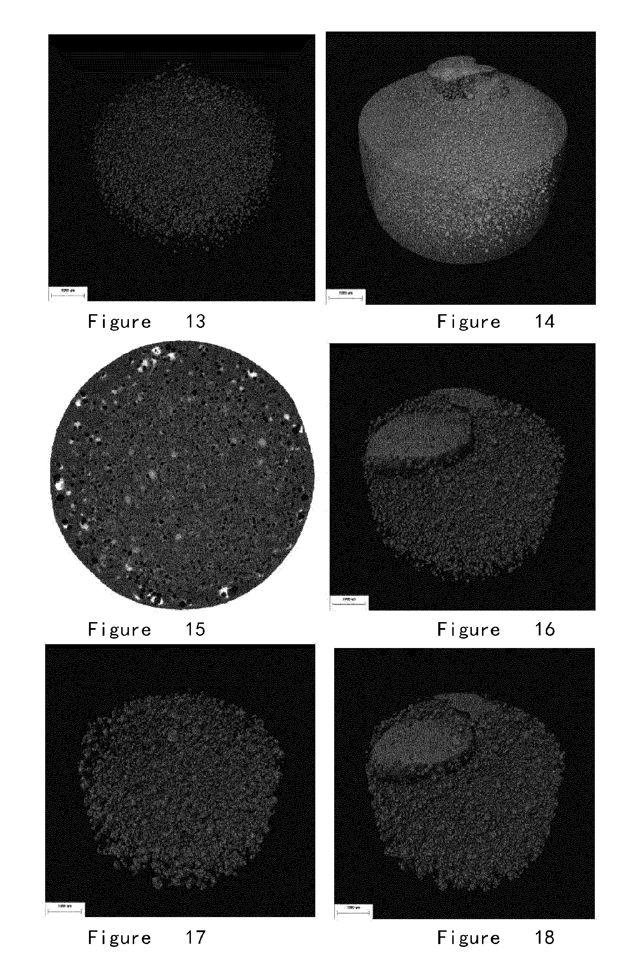

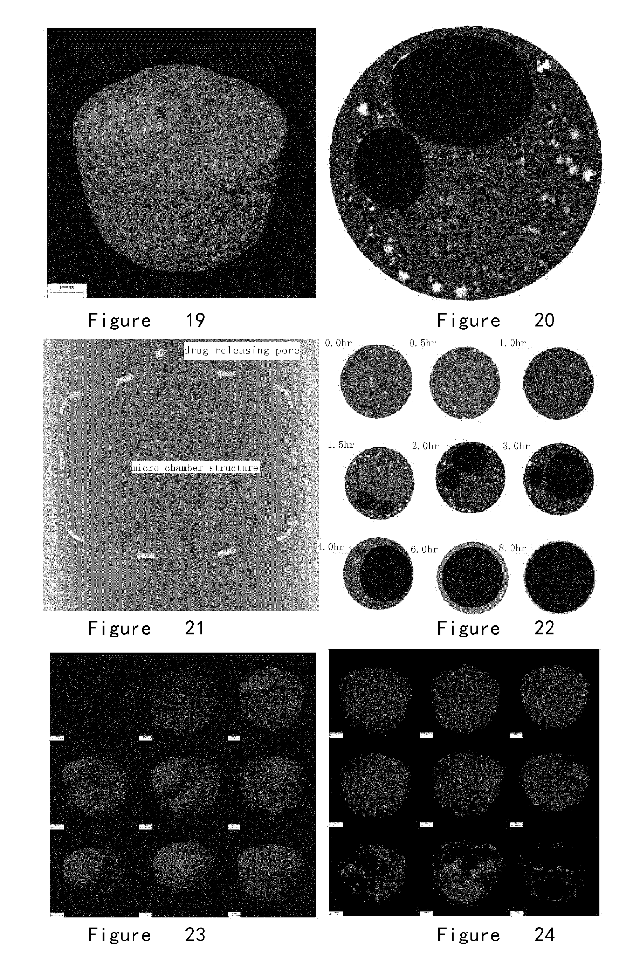

(5) Result: The structure formed according to this formulation is spherical with certain evolution rate. The chamber structures have lager volumes, evolve faster and are easily to fuse into lager chambers. Figure 15 and Figure 20 show the micro chamber structures formed in the osmotic pump tablet after 1.0 hour and 2.0 hours dissolution.

(6)Conclusion: At the earlier stage of drug releasing, micro bubble-shaped chamber structures were formed inside the osmotic pump from the film to the center, and some of micro bubble-shaped chamber structures with small size moved along the coating film towards the drug releasing pore (Figure 21). Evolution rules during the releasing process of the chamber can be seen from time series tomography 2-D images (Figure 22) and 3-D structure images (Figures 23, 24, 25 and 26): at 0.5hr, the core was preliminarily hydrated, and the chamber has higher homogeneity and lower standard deviation of diameter. Most chambers distributed in the hydrated part firstly contacting with dissolution medium around the core. Upon water absorption, the system swelled, pressure was released through drug releasing micro pore, and the formed micro vesicles containing drugs were excreted for drug releasing. At 1.0 hr, the numbers of chambers changed little while the diameter and the homogeneity increased. Most chambers with larger diameter distributed in the highly hydrated part thoroughly contacting with the medium around the core, and a large number of chambers with lower diameters emerged in the core center. At 1.5 hr, the number of chambers started to decline. The existing chambers augmented continuously and then fused into larger chambers. The chamber diameters increased remarkably and the chamber homogeneity decreased significantly compared with that at the time of 1.0 hr. The chamber still had higher sphericity and the chambers with larger diameter still distributed in the highly hydrated part thoroughly contacting with medium around the core. At 2.0 hr, the number of chamber continuously declined, and the existing big chambers continuously swelled and merged the surrounded small chambers and expanded to the core center. The chambers moved towards to the core center gradually with its homogeneity decreased. At 3.0 hr, the core was essentially hydrated, small chambers in edge region drastically reduced, and the chambers essentially expanded to the core center and connecting with releasing medium through drug releasing micro pore, when the releasing entered a dominant stage of corrosion and diffusion. At 4.0 hr, the number of chamber remarkably reduced, and most parts of the core region were occupied. At 6.0 hr, only one chamber was remained occupying most parts of the core region, which had higher sphericity. At 8.0 hr, the remaining chamber further expanded to be more spherical.

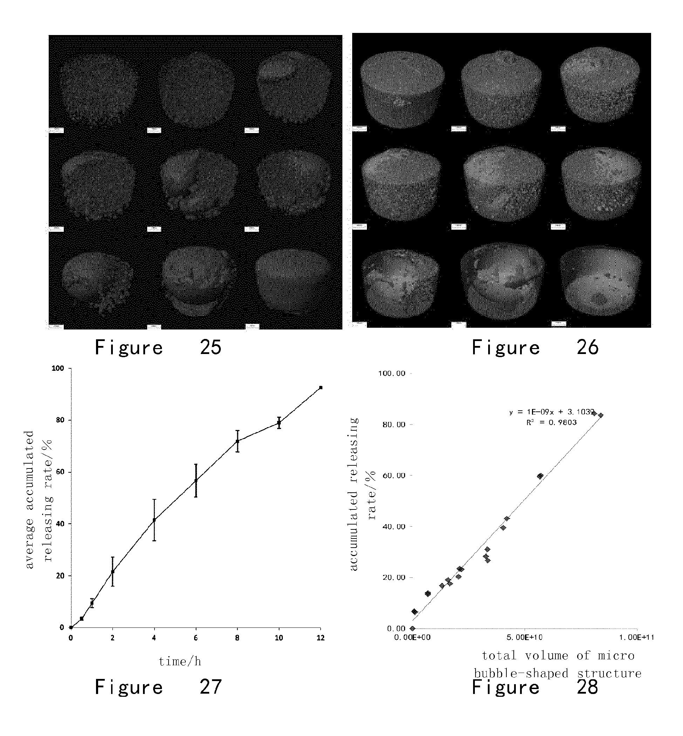

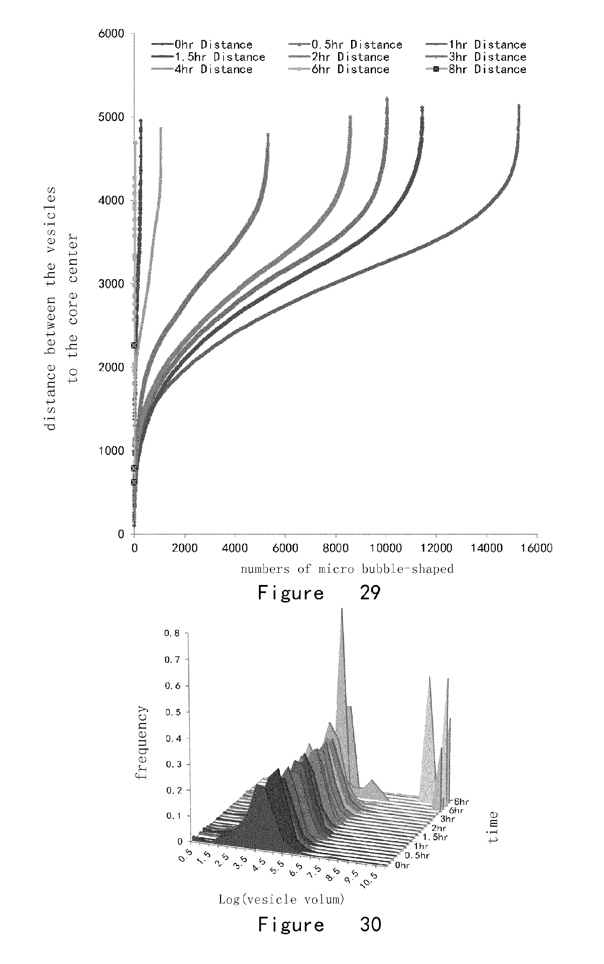

For insoluble drug ketoprofen, copovidone as micro bubble-shaped structure forming material in combination with sodium phosphate as bubble-shaped structure modulating agent were used to modulate the micro structures and the evolution rate thereof for achieving the controlled release of ketoprofen. Figure 27 shows the accumulated releasing rate profile in vitro within 0-12 hours for ketoprofen. It can be seen that drug releases at a steady rate, and the average accumulated releasing rate at 12th hour is over 95%. Furthermore, changes of internal structures of micro chamber over time (Figures 28, 29 and 30) and the total volume of vesicular structures are highly proportional to the drug releasing rate.

**Example 5:**

(1) Core formulation:

| components | mg / tablet |
| --- | --- |
| sodium chloride | 20 |
| copovidone | 60 |

(2) Formulation for coating solution of the semi-permeable film:

| components | amount |
| --- | --- |
| cellulose acetate | 28 g |
| acetone | 2000 mL |

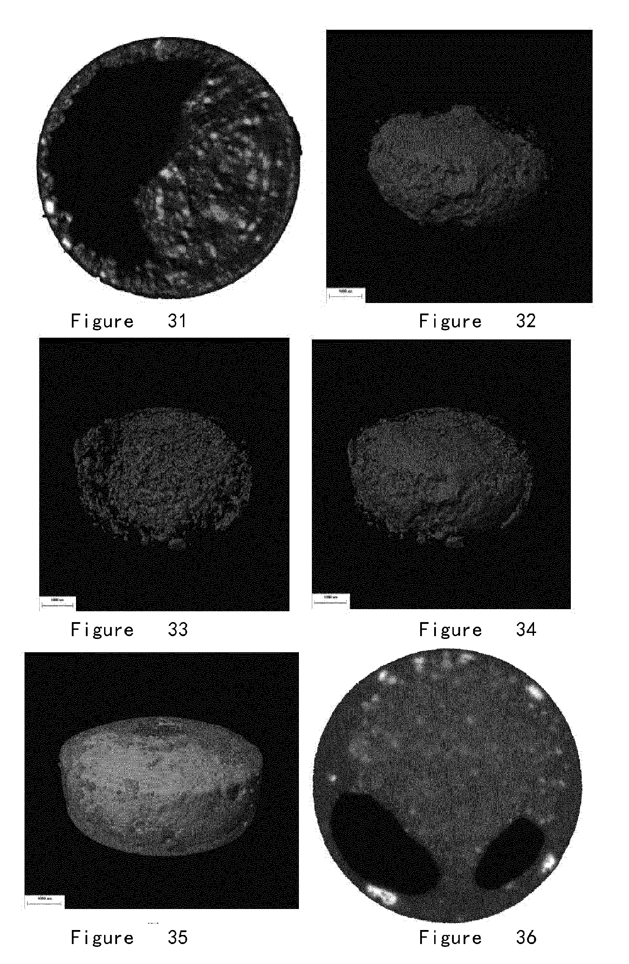

(3) Preparation process: Ditto to Example 2 except that sodium chloride was mixed with copovidone to homogenous and then tableted directly.
(4) Determination method: Ditto to Example 2
(5) Result: Irregular micro chamber structures were formed inside the osmotic pump preparation according to this formulation, which allowed the internal chamber space to connect with the releasing medium outside rapidly, thereby achieving the fast release of the drug in the core. Internal chamber structures of osmotic pump tablet after 1.0-hour dissolution (Figures 31, 32, 33, 34 and 35) show that the chambers were formed very fast while merging, and the surface area of which is large. According to the tomography image, it can be obviously seen that only one irregular chamber was located at one side of the core.
(6) Conclusion: The shape and the evolution rate of the chambers can be modulated to achieve different drug releasing performance by adding different bubble-shaped structure modulating agents.

**Example 6:**

(1) Core formulation:

| Components | mg / tablet |
| --- | --- |
| Copovidone | 57.8 |
| Ketoprofen | 50 |
| sodium phosphate | 15.7 |
| magnesium stearate | 1.2 |

(2) Formulation for coating solution of the semi-permeable film:

| Components | dosage |
| --- | --- |
| cellulose acetate | 28g |
| PEG4000 | 4g |
| Acetone | 2000mL |

(3) Preparation process: Ditto to Example 4
(4) Determination method: Ditto to Example 2
(5) Result: The increase in the content of sodium phosphate content accelerated the hydration and dissolving process of copovidone. Chambers with larger volume were easy to form due to the increase in surface tension of the system and the releasing rate of the drug was relatively increased at the same time. Figures 36, 37, 38, 39, 40 illustrate the internal micro chamber structures of the osmotic pump tablet after 1.0-hour dissolution. It can be obviously seen that two relatively regular chambers with lager size were located on the edge of the core, and some regular micro chambers distributed homogeneously in the other region of the core as well.
(6) Conclusion: The size of the vesicular structures can be increased and the evolution rate can be accelerated by adding the amount of bubble-shaped structure modulating agent in the formulation to rapidly form larger central chamber for connecting releasing medium through drug releasing micro pore and enhancing the drug releasing rate.

**Example 7:**

(1) Core formulation:

| Components | mg / tablet |
| --- | --- |
| polyoxyethylene N10 | 58 |
| ketoprofen | 50 |
| sodium phosphate | 10.8 |
| magnesium stearate | 1.2 |

(2) Formulation for coating solution of the semi-permeable film:

| components | amount |
| --- | --- |
| cellulose acetate | 28 g |
| PEG4000 | 4 g |
| acetone | 2000 mL |

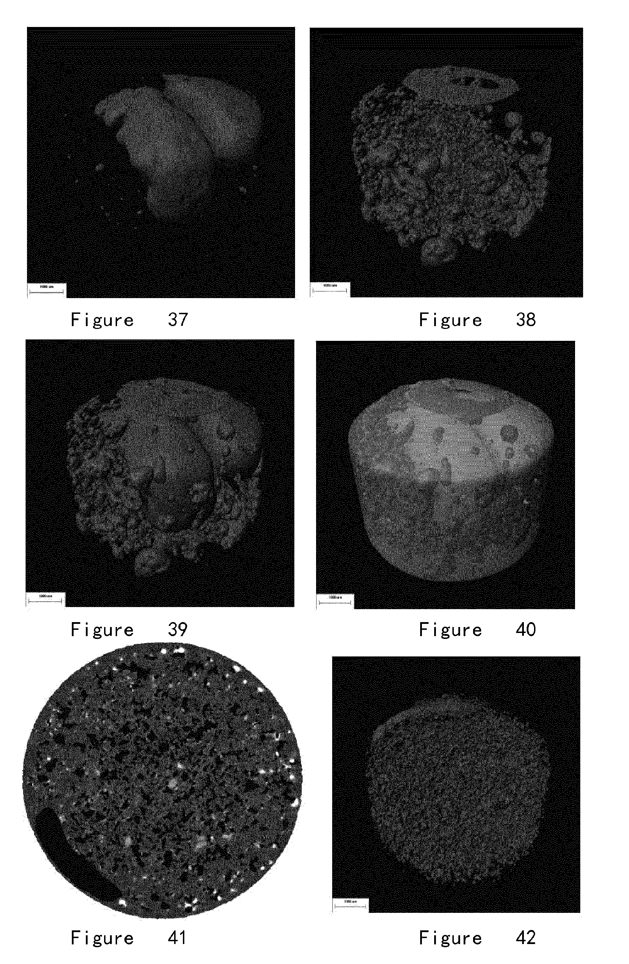

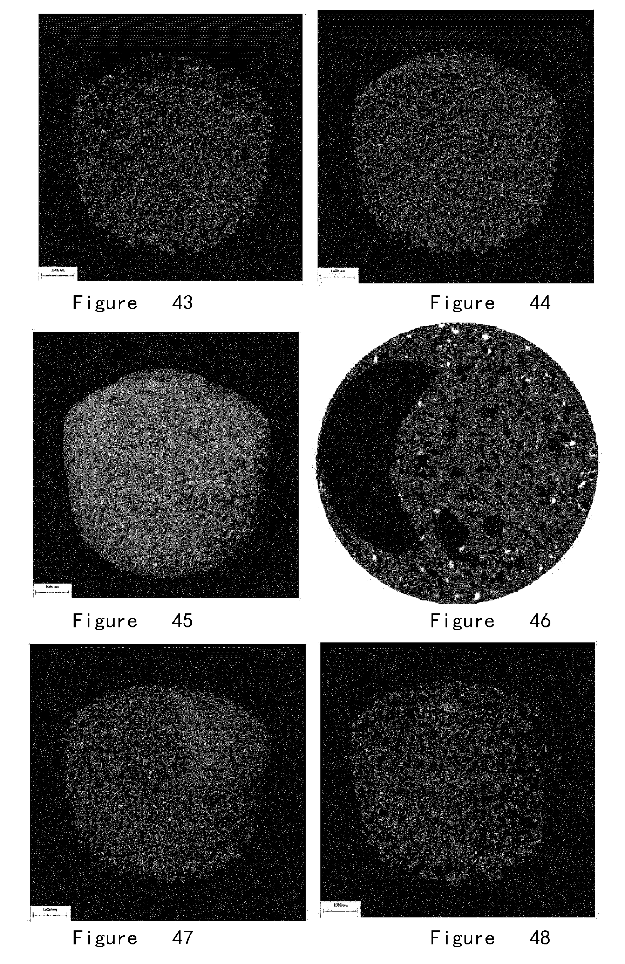

(3) Preparation process: Ditto to Example 4 except that copovidone was replaced by polyoxyethylene.
(4) Determination method: Ditto to Example 2
(5) Result: Figures 41, 42, 43, 44, 45 and Figures 46, 47, 48, 49 and 50 show the chamber structures of the osmotic pump tablet after 1.0-hour and 2.0-hours dissolution respectively. These micro chambers with irregular shapes distributed in the whole internal core. Chamber fusing can be seen over time.

(6) Conclusion: The shapes and evolution modes can be modulated by selecting bubble-shaped forming materials with different solubility, hydration speed and system viscosity to control the portions excreted, dissolved and corroded from the swelled vesicular during the releasing process for achieving different drug releasing characteristics.

**Example 8:**

(1) Core formulation:

| components | mg / tablet |
| --- | --- |
| lactose | 58 |
| ketoprofen | 50 |
| sodium phosphate | 10.8 |
| magnesium stearate | 1.2 |

(2) Formulation for coating solution of the semi-permeable film:

| components | dosage |
| --- | --- |
| cellulose acetate | 28 g |
| PEG4000 | 4 g |
| acetone | 2000 mL |

(3) Preparation process: Ditto to Example 4 except that copovidone is replaced by lactose.

(4) Determination method: Ditto to Example 2.

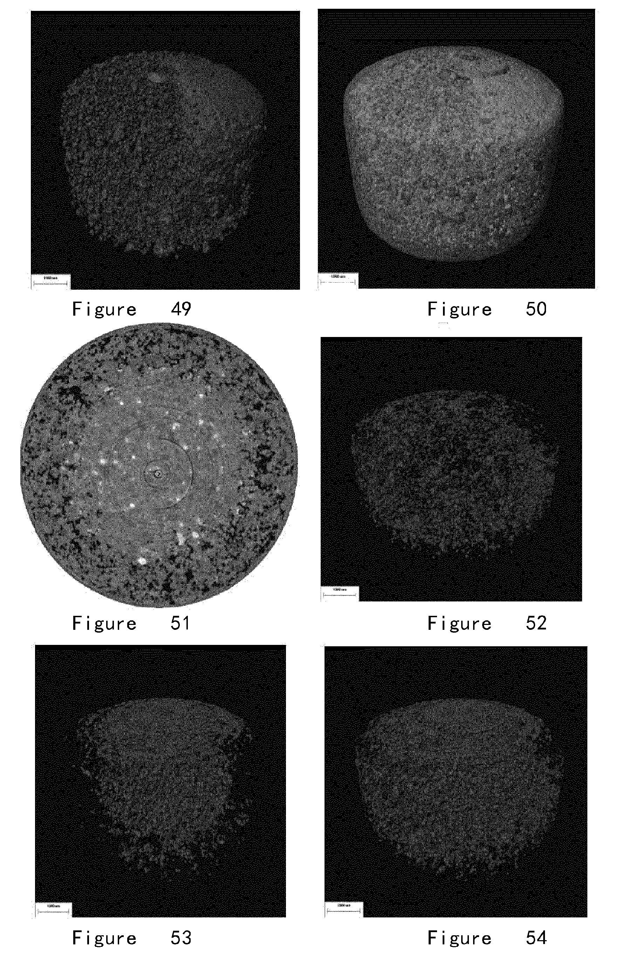

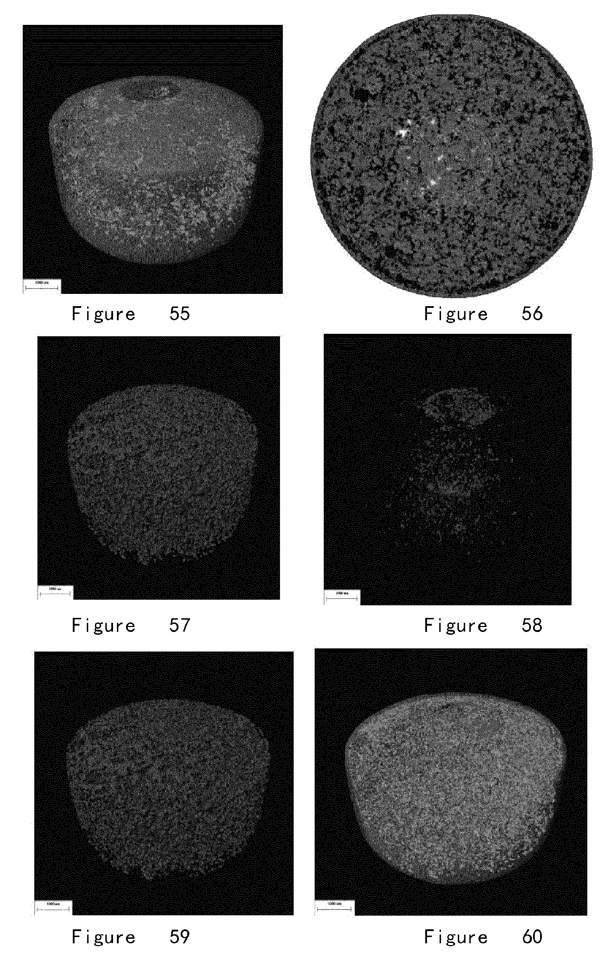

(5) Result: Figures 51, 52, 53, 54, 55 and Figures 56, 57, 58, 59, 60 respectively show the chamber structures of osmotic pump tablet after 1.0-hour and 2.0-hour dissolution. Micro chamber structures with irregular shapes inside the core were firstly formed around the semi-permeable coating film. As time went by, the number of chamber increased rapidly at the early stage, and volume of single irregular chamber changed little so that numerous micro chambers distributed in the whole internal core with a relatively homogenous manner.

(6) Conclusion: Water soluble non-high molecular materials as bubble-shaped structure forming materials in combination with chamber structure modulating agents will remarkably increase the forming rate of bubble-shaped structures at early releasing stage and increase drug releasing rate.

**Example 9:**

(1) Core formulation:

| components | mg / tablet |
| --- | --- |
| copovidone | 58 |
| ketoprofen | 50 |
| sodium chloride | 10.8 |
| magnesium stearate | 1.2 |

(2) Formulation for coating solution of the semi-permeable film:

| components | dosage |
| --- | --- |
| cellulose acetate | 28g |
| PEG4000 | 4g |
| acetone | 2000mL |

(3) Preparation process: Ditto to Example 4 except that sodium phosphate is replaced by sodium chloride.

(4) Determination method: Ditto to Example 2

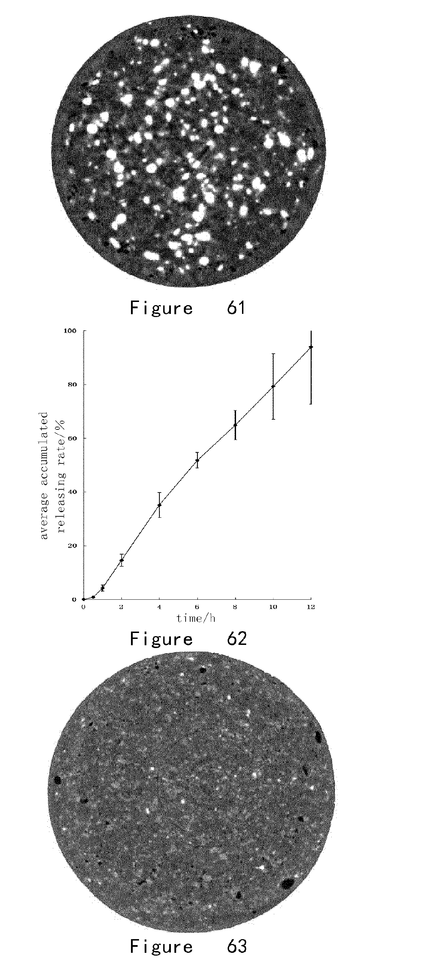

(5) Result: Figure 61 shows the chamber structure of osmotic pump tablet after 1.0-hour dissolution. Fine chambers can be observed in the figure, which distributed around the film edge with poor sphericity and uniformity. Some of small chambers had assembled and merged.

(6) Conclusion: Water soluble non-high molecular materials as bubble-shaped structure forming materials in combination with chamber structure modulating agents will remarkably increase the forming rate of bubble-shaped structures at early releasing stage and increase drug releasing rate.

**Example 10:**

(1) Core formulation:

| components | mg / tablet |
| --- | --- |
| azithromycin | 50 |
| Citric acid | 14 |
| copovidone-S630 | 48.8 |
| talcum powder | 6 |
| magnesium stearate | 1.2 |

(2) Formulation for coating solution of the semi-permeable film:

| components | dosage |
| --- | --- |
| cellulose acetate | 28g |
| PEG4000 | 4g |
| acetone | 2000mL |

(3) Preparation process: Sifted azithromycin and copovidone-S630 were weighed according to the formulation and then. Then, citric acid as listed in the formulation was added and mixed to homogeneous, and finally, a suitable amount of talcum power and magnesium stearate were added. The product was mixed to homogeneous and tableted. The core was coated by cellulose acetate-PEG4000 solution in acetone till the weigh increasing of the core reached 8%. A pore with a diameter of 0.8 mm on one side of the coating film was punched by laser or mechanic means.

(4) Determination method: According to Method 1 of Releasing Assay in Chinese Pharmacopoeia 2010, releasing rates at different times were determined by using 900 mL water as solvent at 100 RPM.

(5) Result: The *in vitro* time-accumulated releasing curve of azithromycin (Figure 62) shows that azithromycin released thoroughly with zero-order releasing characteristics in 12 hours, and the average accumulated releasing rate at 12th hour is over 95%. Figure 63 demonstrates the chamber structures of the osmotic pump tablet after 1.0-hour dissolution. Numerous chambers distributed in the whole internal core homogeneously with good uniformity and sphericity.

(6) Conclusion: For different drugs, different bubble-shaped structure forming materials and different chamber structure modulating agents can be selected to modulate the 3-D shapes, evolution modes and evolution speed of chamber so as to achieve the object of controlled drug releasing.

**Example 11:**

(1) Core formulation:

| components | mg / tablet |
| --- | --- |
| captopril | 50 |
| sodium chloride | 6 |
| copovidone-S630 | 61.6 |
| Vitamin C | 1.2 |

(continued)

| components | mg / tablet |
| --- | --- |
| magnesium stearate | 1.2 |

(2) Formulation for coating solution of the semi-permeable film:

| components | amount |
| --- | --- |
| cellulose acetate | 28g |
| PEG4000 | 7g |
| acetone | 2000mL |

(3) Preparation process: Captopril and copovidone-S630 sifted by a 100-mesh sifter were weighed according to the formulation and mixed to homogeneous. Then, sodium chloride and Vitamin C as listed in the formulation were added and mixed to homogeneous. Finally, a suitable amount of magnesium stearate was added and then the product was mixed to homogeneous and tableted. The rest steps are ditto to Example 10.

(4) Determination method: Ditto to Example 10.

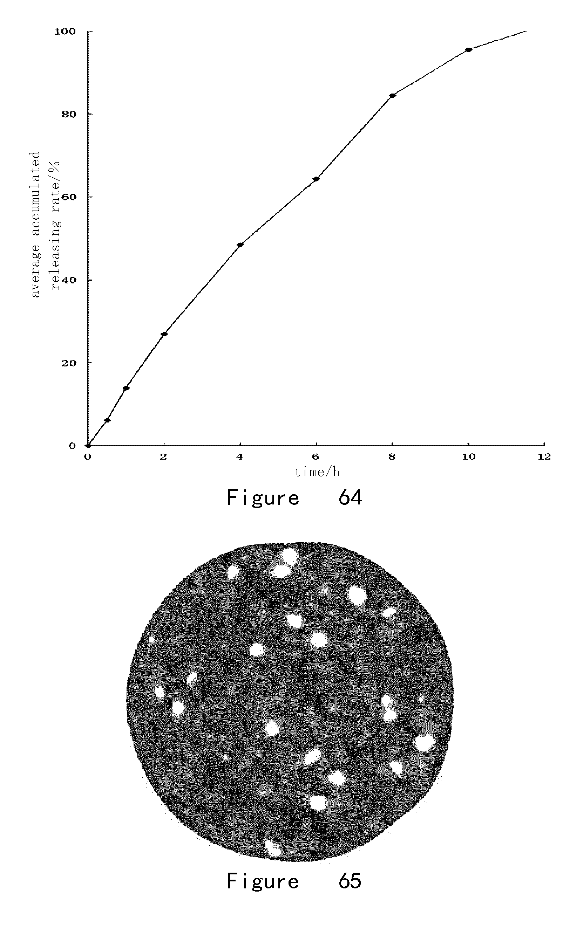

(5) Result: The *in vitro* time-accumulated releasing curve of captopril (Figure 64) shows that captopril released thoroughly with zero-order releasing characteristics in 12 hours, and the average accumulated releasing rate at 12th hour is approaching 100%. Figure 65 demonstrates the chamber structures of the osmotic pump tablet after 1.0-hour dissolution. Fine chambers distributed along the core edge homogeneously with good uniformity and sphericity.

(6) Conclusion: For different drugs, different bubble-shaped structure forming materials and different chamber structure modulating agents can be selected to modulate the 3-D shapes, evolution modes and evolution speed of chamber so as to achieve the object of controlled drug releasing.

**Claims**

1. A drug osmotic pump preparation comprising:

   a core, comprising drug(s) (or drug active ingredient(s)) and chamber structure forming material(s); and
   a coating film for covering the core with drug releasing pore(s) set on said coating film;
   wherein, when the drug osmotic pump preparation is used for drug release in water or aqueous medium, micro chamber structures are thus formed in the coating film by the chamber structure forming materials and total volume of the micro chamber structures Vb increases with releasing time, and releasing rate R of drug active ingredients (or releasing amount A) is directly proportional to Vb.

2. The osmotic pump preparation according to claim 1, wherein ratio between maximum value of Vb, namely Vbmax and core volume Va is 90-100: 100, preferably 95-100: 100.

3. The osmotic pump preparation according to claim 1, wherein said releasing rate R (or releasing amount A) is directly proportional to Vb refers to "the correlation coefficient between release rate R (releasing amount A) and Vb is $\geq 0.9$, preferably $\geq 0.95$".

4. The osmotic pump preparation according to claim 1, wherein said micro chamber structures are liquid chamber structures.

5. The osmotic pump preparation according to claim 1, wherein said core contains the following components:

   0.2-99 % , preferably 1-60 wt% of drug(s);
   15-99.8 wt%, preferably 20-95wt % , more preferably 30-90 wt% of chamber structure forming material(s);
   0-50wt%, preferably 1-50wt% of chamber structure shape-modulating agent(s);
   0-10wt%, preferably 0.5-10 wt% of lubricating agent(s); and
   0-60wt%, 1-60 wt% of pharmaceutical acceptable auxiliary material(s); wherein the content is based on total weight of the core.

6. The osmotic pump preparation according to claim 1 or 5, wherein said chamber structure forming material is one or more selected from a group consisting of povidone, polyoxyethylene, copovidone and lactose.

7. The osmotic pump preparation according to claim 1 or 5, wherein said chamber structure shape-modulating agent is acid or basic material or salts thereof.

8. The drug osmotic pump preparation according to claim 7, wherein said acid or basic material or salts thereof is one or more selected from a group consisting of sodium phosphate, disodium hydrogen phosphate, sodium dihydrogen phosphate, sodium chloride, potassium chloride, sodium hydroxide, amino acid, citric acid and tartaric acid.

9. The drug osmotic pump preparation according to claim 1 or 2, wherein said drug is water soluble drug or water insoluble drug.

10. The drug osmotic pump preparation according to claim 9, wherein said water soluble drug is captopril, ribavirin, ketorolac tromethamine, levocarnitine, pentoxifylline, salvianolic acid, doxofylline, metformin, alfuzosin hydrochloride, ligustrazine phosphate, diltiazem hydrochloride, lamivudine or salbutamol sulfate; and / or
said water insoluble drug is ibuprofen, ketoprofen, azithromycin, roxithromycin, glipizide, felodipine, nimodipine, nisoldipine, nitrendipine, nifedipine, doxazosin mesylate, risperidone, famotidine, huperzine a, gliquidone, simvastatin, ambroxol hydrochloride, barnidipine, etodolac, acipimox, indapamide, azelnidipine, nimesulide, levetiracetam, tamsulosin hydrochloride, pitavastatin calcium, or etodolic acid.

11. A method for designing a target preparation with desired drug releasing rate or releasing curve, comprising the steps of:

   (i) providing a drug osmotic pump preparation according to claim 1, wherein said preparation comprises said drug as an active ingredient;
   (ii) placing said drug osmotic pump preparation into water or aqueous solvent, and detecting the evolution situation of the micro chamber structures in said drug osmotic pump preparation;
   (iii) calculating releasing rate or releasing curve of the drug according to the detected evolution situation of the micro chamber structures;
   (iv) selecting a drug osmotic pump preparation as a target preparation from the detected preparations, the releasing rate or releasing curve of which is close to or in accordance with the desired releasing rate or releasing curve of the drug.

12. The method according to claim 11, wherein step (iv) further comprises:

   based on the detected releasing rate or releasing curve, selecting a preparation A, the releasing rate or releasing curve of which is close to that of the desired drug, modulating the formulation according to preparation A and repeating step (i), (ii) and (iii) for one or more times, thereby selecting a drug osmotic pump preparation as the target preparation from the detected preparations, the releasing rate or releasing curve of which is closer to or in accordance with desired releasing rate or releasing curve of the drug.

13. A preparation method for drug osmotic pump preparation according to claim 1, comprising the steps of:

   providing a core, wherein said core comprises drug(s) (or drug active ingredient(s)) and chamber structure forming material(s);
   coating said core, to form a coating film covering said core;
   punching said coating film, to form drug releasing pore on said coating film.

14. A drug osmotic pump preparation comprising core and coating film,
based on the total weight of the core, said core comprises: 1-60 wt% of drug(s), 5-90 wt% of chamber structure forming material(s), 1-50 wt% of chamber structure shape-modulating agent(s), 0.5-10 wt% of lubricating agent(s), and 1-60 wt% of pharmaceutical acceptable auxiliary material(s);
based on the total weight of the coating film, said coating film comprises: 40-90 wt% of semi-permeable film coating material(s), 5-40 wt% of pore forming agent(s), and 0-20 wt% of plasticizer(s).

15. The drug osmotic pump preparation according to claim 14, wherein, it comprises
a core comprising drug(s) (or drug active ingredient(s)) and chamber structure forming material(s); and

a coating film for covering the core with drug releasing pore(s) on said coating film;

wherein, when the drug osmotic pump preparation releases drug in water or aqueous medium, micro chamber structures are thus formed in the coating film by the chamber structure forming material(s) and total volume of the micro chamber structure $V_b$ increases with releasing time, and release rate R of the drug active ingredient(s) (or releasing amount A) is directly proportional to $V_b$.

Figure 1

Figure 2

Figure 3

Figure 4

Figure 5

Figure 6

Figure 7

Figure 8

Figure 9

Figure 10

Figure 11

Figure 12

Figure 13

Figure 14

Figure 15

Figure 16

Figure 17

Figure 18

Figure    19

Figure    20

drug releasing pore

micro chamber structure

Figure    21

0.0hr    0.5hr    1.0hr

1.5hr    2.0hr    3.0hr

4.0hr    6.0hr    8.0hr

Figure    22

Figure    23

Figure    24

Figure 25

Figure 26

Figure 27

Figure 28

Figure 29

Figure 30

Figure    31

Figure    32

Figure    33

Figure    34

Figure    35

Figure    36

Figure     37

Figure     38

Figure     39

Figure     40

Figure     41

Figure     42

Figure 43

Figure 44

Figure 45

Figure 46

Figure 47

Figure 48

Figure 49

Figure 50

Figure 51

Figure 52

Figure 53

Figure 54

Figure　55

Figure　56

Figure　57

Figure　58

Figure　59

Figure　60

Figure 61

Figure 62

Figure 63

Figure    64

Figure    65

## INTERNATIONAL SEARCH REPORT

| | International application No. |
|---|---|
| | PCT/CN2013/001256 |

### A. CLASSIFICATION OF SUBJECT MATTER

See the extra sheet

According to International Patent Classification (IPC) or to both national classification and IPC

### B. FIELDS SEARCHED

Minimum documentation searched (classification system followed by classification symbols)

IPC: A61K

Documentation searched other than minimum documentation to the extent that such documents are included in the fields searched

Electronic data base consulted during the international search (name of data base and, where practicable, search terms used)
CNABS, CPRSABS, CNMED, MOABS, HKABS, TWABS, TWMED, CNKI, google scholar, osmotic pump, zhang jiwen, tablet+, cavity, pore, volume, formulation, process, prepara+

### C. DOCUMENTS CONSIDERED TO BE RELEVANT

| Category* | Citation of document, with indication, where appropriate, of the relevant passages | Relevant to claim No. |
|---|---|---|
| PX | CN 102871982 A (CHINESE ACAD SCI SHANGHAI MATERIAL MEDIC) 16 January 2013 (16.01.2013) see embodiment 1 | 1-10, 13-15 |
| X | CN 101095681 B (SHENYANG YAODA PREPARATION NEW TECHNOLOGY CO LTD) | 1-10,13 |
| Y | 20 April 2011(20.04. 2011) see embodiment 1 | 14-15 |
| Y | see claim 4 | 14-15 |
| X | CN 101744787 A (POISON MEDICINE INST PLA MILITARY MEDICA) 23 June 2010 (23.06.2010) see embodiment 6 | 1-10, 13-15 |
| X | CN 101269049 B (GUANGDONG PHARM COLLEGE) 25 January 2012 (25.01.2012) see embodiment 1 | 1-10, 13-15 |
| X | CN 101953822 A (HEFEI LIFANG PHARM CO LTD) 26 January 2011 (26.01.2011) see embodiment 4 | 1-10, 13-15 |
| X | CN 102626428 A (XUZHOU MEDICAL COLLEGE) 08 August 2012 (08.08.2012) see embodiment 1 | 1-10, 13-15 |

☒ Further documents are listed in the continuation of Box C.    ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 10 January 2014 (10.01.2014) | 30 January 2014 (30.01.2014) |

| Name and mailing address of the ISA State Intellectual Property Office of the P. R. China No. 6, Xitucheng Road, Jimenqiao Haidian District, Beijing 100088, China Facsimile No. (86-10) 62019451 | Authorized officer TIAN, Xiaoming Telephone No. (86-10) 62411130 |
|---|---|

Form PCT/ISA /210 (second sheet) (July 2009)

## INTERNATIONAL SEARCH REPORT

| International application No. |
| --- |
| PCT/CN2013/001256 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | JP 2003-267861 A (KOREA RES INST CHEM TECHNOLOGY) 25 September 2003 (25.09.2003) see the whole document | 1-10, 13-15 |
| A | CN 101167701 A (CHINESE ACAD SCI SHANGHAI MATERIAL MEDIC) 30 April 2008 (30.04.2008) see the whole document | 1-10, 13-15 |
| A | US 4203439 A (ALZA CORP.) 20 May 1980 (20.05.1980) see the whole document | 1-10, 13-15 |
| A | HAIYAN LI, et al. Microstructural investigation to the controlled release kinetics of monolith osmotic pump tablets via synchrotron radiation X-ray microtomography. INTERNATIONAL JOURNAL OF PHARMACEUTICS. 10 May 2012, Vol. 427, No.2, pp. 270-275, ISSN 0378-5173 | 1-10,13-15 |
| PA | XIANZHEN YIN, et al. Fractal structure determines controlled release kinetics of monolithic osmotic pump tablets. JOURNAL OF PHARMACY AND PHARMACOLOGY. July 2013, Vol. 65, No.7, pp. 953-959, ISSN 0022-3573, Online ISSN 2042-7158 | 1-10,13-15 |
| PA | YANG SHUO, et al. Research progress on architecture of dosage forms using synchrotron radiation X-ray microtomography. CHINESE BULLETION OF LIFE SCIENCES. August 2013, Vol. 25, No. 8, pp. 794-802, ISSN 1004-0374 | 1-10,13-15 |

Form PCT/ISA/210 (continuation of second sheet) (July 2009)

## INTERNATIONAL SEARCH REPORT

| International application No. |
|---|
| PCT/CN2013/001256 |

**Box No. II    Observations where certain claims were found unsearchable (Continuation of item 2 of first sheet)**

This international search report has not been established in respect of certain claims under Article 17(2)(a) for the following reasons:

1. ☒    Claims Nos.:11-12
        because they relate to subject matter not required to be searched by this Authority, namely:
        Claims 11-12 are directed to a designed method of target preparation with desired drug release rate or desired drug release profile, said subject matter sets the target preparation with desired release characteristics, uses set measuring and calculating method and choosing the product achieved the setting goal. The subject matter of claims 11-12 doesn't use technical means or utilize natural law, neither solves technical problems or has a technical effect. The subject matter of claims 11-12 therefore relates to rules or methods of performing mental acts and thus does not warrant an international search according to the criteria set out in Rule 39. 1(iii).

2. ☐    Claims Nos.:
        because they relate to parts of the international application that do not comply with the prescribed requirements to such an extent that no meaningful international search can be carried out, specifically:

3. ☐    Claims Nos.:
        because they are dependent claims and are not drafted in accordance with the second and third sentences of Rule 6.4(a).

**Box No. III   Observations where unity of invention is lacking (Continuation of item 3 of first sheet)**

This International Searching Authority found multiple inventions in this international application, as follows:

1. ☐    As all required additional search fees were timely paid by the applicant, this international search report covers all searchable claims.

2. ☐    As all searchable claims could be searched without effort justifying additional fees, this Authority did not invite payment of additional fees.

3. ☐    As only some of the required additional search fees were timely paid by the applicant, this international search report covers only those claims for which fees were paid, specifically claims Nos.:

4. ☐    No required additional search fees were timely paid by the applicant. Consequently, this international search report is restricted to the invention first mentioned in the claims; it is covered by claims Nos.:

**Remark on protest**        ☐    The additional search fees were accompanied by the applicant's protest and, where applicable, the payment of a protest fee.

                ☐    The additional search fees were accompanied by the applicant's protest but the applicable protest fee was not paid within the time limit specified in the invitation.

                ☐    No protest accompanied the payment of additional search fees.

Form PCT/ISA /210 (continuation of first sheet (2)) (July 2009)

## INTERNATIONAL SEARCH REPORT
### Information on patent family members

| International application No. |
|---|
| PCT/CN2013/001256 |

| Patent Documents referred in the Report | Publication Date | Patent Family | Publication Date |
|---|---|---|---|
| CN 102871982 A | 16.01.2013 | None | |
| CN 101095681 B | 20.04.2011 | CN 101095681 A | 02.01.2008 |
| CN 101744787 A | 23.06.2010 | CN 101744787 B | 27.03.2013 |
| CN 101269049 B | 25.01.2012 | CN 101269049 A | 24.09.2008 |
| CN 101953822 A | 26.01.2011 | CN 101953822 B | 30.05.2012 |
| CN 102626428 A | 08.08.2012 | None | |
| JP 2003-267861 A | 25.09.2003 | KR 20030073579 A | 19.09.2003 |
| | | KR 453288 B | 21.10.2004 |
| CN 101167701 A | 30.04.2008 | None | |
| US 4203439 A | 20.05.1980 | None | |

Form PCT/ISA /210 (patent family annex) (July 2009)

**INTERNATIONAL SEARCH REPORT**

International application No.

PCT/CN2013/001256

Continuation of Part A of the second sheet

A61K 9/36 (2006.01) i

A61K 9/32 (2006.01) i

A61K 9/22 (2006.01) i

Form PCT/ISA /210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5221278 A
- US 6132420 A
- US 7338663 B
- US 20036596314 A
- US 4008719 A
- US 6899887 B
- US 4203439 A
- US 4331728 A

**Non-patent literature cited in the description**

- **L. LIU et al.** *J Control. Release,* 2000, vol. 68, 145-156
- **D. PRABAKARAN et al.** *Int. J. Pharm.,* 2004, vol. 284, 95-108